# EUROPEAN PATENT SPECIFICATION

(11) **EP 1 893 273 B1**
(45) Date of publication and mention of the grant of the patent: **25.06.2014**
(21) Application number: 06824753.5
(22) Date of filing: 18.05.2006
(51) Int. Cl.: A61M 16/20, F16K 15/14

(54) **ADAPTER FOR USE WITH AEROSOLIZATION DEVICE FOR ENDOBRONCHIAL THERAPY**
ADAPTER ZUR ANWENDUNG MIT EINER AEROSOLISIERUNGSVORRICHTUNG FÜR ENDOBRONCHIALE THERAPIE
ADAPTATEUR POUR L'UTILISATION AVEC UN DISPOSITIF D'AÉROSOLISATION POUR TRAITEMENT ENDOBRONCHIQUE

(30) Priority: 18.05.2005 US 682099 P; 29.09.2005 US 722637 P
(43) Date of publication of application: 05.03.2008
(73) Proprietor: Nektar Therapeutics, San Carlos, CA 94070 (US)
(72) Inventor: CECKA, Walter, Berkeley, CA 94705 (US); ALSTON, William, W., San Jose, CA 95124 (US); SMITH, Adrian, E., Emerald Hills, CA 94062 (US); HALL, Gregory, W., Redwood City, CA 94063 (US); FISHER, Winfield, Castro Valley, CA 94546 (US); COHEN, Gal, San Francisco, CA 94110 (US)
(74) Representative: Boult Wade Tennant
(86) International application number: PCT/US2006/019446
(87) International publication number: WO 2007/030162

(56) References cited:
- WO-A-00/12161
- WO-A-98/20938
- WO-A-98/20938
- WO-A1-02/092169
- WO-A1-02/092169
- AU-B2- 496 203
- AU-B2- 496 203
- GB-A- 2 219 844
- GB-A- 2 219 844
- US-A- 4 259 951
- US-A- 4 259 951
- US-A- 4 428 392
- US-A- 4 428 392
- US-A- 4 796 614
- US-A- 5 099 833
- US-A- 5 451 190
- US-A- 5 666 946
- US-A- 5 865 171
- US-A1- 2005 039 746
- US-B1- 6 631 721
- US-B1- 6 631 721

## Description

### BACKGROUND OF THE INVENTION

### Field of the Invention

Embodiments of the present invention relate to adapters for introducing aerosols into a patient in need of such introduction, and more particularly to adapters for ventilator circuits, for nebulizers, and for introducing aerosols into ventilator circuits and/or into a patient in need of such introduction.

### Background Art

The need for effective therapeutic treatment of patients has resulted in the development of a variety of pharmaceutical formulation delivery techniques. One traditional technique involves the oral delivery of a pharmaceutical formulation in the form of a pill, capsule, elixir, or the like. However, oral delivery can in some cases be undesirable. For example, many pharmaceutical formulations may be degraded in the digestive tract before the body can effectively absorb them. Inhaleable drug delivery, also known as pulmonary delivery, where a patient orally or nasally inhales an aerosolized pharmaceutical formulation to deliver the formulation to the patient's respiratory tract, may also be effective and/or desirable. In some inhalation techniques, an aerosolized pharmaceutical formulation provides local therapeutic treatment and/or prophylaxis to a portion of the respiratory tract, such as the lungs, to treat respiratory diseases such as asthma and emphysema and/or to treat local lung infections, such as fungal infections and cystic fibrosis. In other inhalation techniques, a pharmaceutical formulation is delivered deep within a patient's lungs where it may be absorbed into the bloodstream for systemic delivery of the formulation throughout the body. Many types of aerosolization devices exist including devices comprising a pharmaceutical formulation stored in or with a propellant, devices that aerosolize a dry powder, devices which use a compressed gas or other mechanism to aerosolize a liquid Pharmaceutical formulation, and similar devices.

One known aerosolization device is commonly referred to as a nebulizer. A nebulizer comprises a container having a reservoir that contains a fluid, liquid, or liquefiable formulation. If liquid, the pharmaceutical formulation generally comprises an active agent that is either in solution or suspended or dispersed within a liquid medium. Energy is introduced into the reservoir to aerosolize the liquid pharmaceutical formulation to allow delivery to the lungs of a patient. In one type of nebulizer, generally referred to as a jet nebulizer, compressed gas is forced through an orifice in the container. The compressed gas forces liquid to be withdrawn through a nozzle, and the withdrawn liquid mixes with the flowing gas to form aerosol droplets. A cloud of droplets is then administered to the patient's respiratory tract. In another type of nebulizer, generally referred to as a vibrating mesh nebulizer, energy such as high frequency ultrasonic waves are generated to vibrate a mesh. This vibration of the mesh aerosolizes the liquid pharmaceutical formulation to create an aerosol cloud that is administered to the patient's lungs. In still another type of nebulizer, ultrasonic waves are generated to directly vibrate and aerosolize the pharmaceutical formulation.

### Aerosolized Particle Devices

The valves, devices, fittings, systems, components and adapters for introducing aerosols into a patient in need of such introduction may also be suitably used with dry-powder administration devices, such as passive dry powder inhalers and active dry powder inhalers. A passive dry powder inhaler comprises an inhalation device which relies upon a patient's inspiratory effort to disperse and aerosolize a pharmaceutical composition contained within the device in a reservoir or in a unit dose form and does not include inhaler devices which comprise a means for providing energy, such as pressurized gas and vibrating or rotating elements, to disperse and aerosolize the drug composition. An active dry powder inhaler comprises to an inhalation device that does not rely solely on a patient's inspiratory effort to disperse and aerosolize a pharmaceutical composition contained within the device in a reservoir or in a unit dose form and does include inhaler devices that comprise a means for providing energy to disperse and aerosolize the drug composition, such as pressurized gas and vibrating or rotating elements.

Nebulizers are often used to deliver (1) an aerosolized pharmaceutical formulation to a hospitalized or non-ambulatory patient; and/or (2) large doses of aerosolized active agent; and/or (3) an aerosolized pharmaceutical formulation to a child or other patient unable to receive a dry powder or propellant based pharmaceutical formulation.

Nebulizers are useful for delivering an aerosolized pharmaceutical formulation to the respiratory tract of a patient who is breathing under the assistance of a ventilator. But there are problems associated with the introduction of aerosolized pharmaceutical formulation into ventilator circuits. For example, by introducing the aerosolized pharmaceutical formulation into the inspiratory line of the ventilator, significant residence volume exists between the point of introduction and the patient's lungs. Accordingly, large amounts of aerosolized pharmaceutical formulation are needled and much of the formulation is lost to the exhalation line. This problem is exacerbated when the nebulizer is used in conjunction with ventilators having continual bias flows. In addition, the large residence volume in the ventilator line may dilute the aerosolized pharmaceutical formulation to an extent where the amount delivered to the patient is difficult to reproduce consistently. Difficulty in reproducing consistent dose is further exacerbated by patient-to-patient variation in ventilator parameters, such as tidal volume, flow rates, etc.

In typical vibrating mesh nebulizers, the mesh is constructed to be part of an integral vibrating mesh assembly, and the liquid to be aerosolized is introduced by simply pouring the liquid into a chamber. The chamber may be arranged to bring by gravity the liquid into contact with the integral mesh. In some cases, a wick is used to bring the liquid into contact with the mesh.

For instance, vibrating mesh nebulizers may be mounted on a T-piece in a ventilator circuit between an inspiratory line and a Y-piece. To administer liquid drug formulation, a cap is opened. Liquid drug formulation is poured into a drug holding chamber where the liquid comes into contact with a vibrating mesh element. Proper electronic signal, which may be a sinusoidal, square or other waveform of specified amplitude and frequency, is delivered via cable connected to cable receptacle to provide electronic signal to vibrating mesh element in order to deliver liquid drug formulation in the form of aerosol into the T- piece and ventilator circuit to the patient.

Typical vibrating mesh nebulizers are subject to repeated use for nebulizing a variety of liquid drug formulations. The mesh therefore comes in contact with these liquid drug formulations, sometimes in concentrated form as the liquid drag formulations may evaporate the solvent carrier. Repeated use can result in further concentration of liquid drug formulations such that the mesh is subject to corrosion. Furthermore, use of multiple liquid drug formulations administered sequentially may result in inadvertent and potentially dangerous cross-contamination of the different liquid drug formulations. For safety, cleaning of vibrating mesh nebulizers is important after each dose to help guard against corrosion and cross-contamination, putting this safety issue into the hands of the healthcare worker administering the liquid drug formulations and requiring that the mesh and related vibrating mesh hardware be designed and manufactured to resist corrosion and other long-term damaging effects of repeated exposure to liquid drug formulations.

US 5,099,833 discloses a medication aerosol delivery system. The system includes a flexible reservoir for receiving medicated aerosol from a nebulizer when a patient is not inhaling. The aerosol in the reservoir can be later inhaled during the next patient inhalation. The threshold filling pressure (the pressure required for aerosol to enter the reservoir) is less than a threshold opening pressure of a one-way valve that is located between the reservoir and the patient. The medication system aims to increase efficiency over other medication systems without a reservoir by allowing medicated aerosol to be collected in a reservoir between patient inhalations.

U.S. Patent No. 3,726,274, discloses a non-rebreathing valve assembly and compression bulb resuscitator using the same. One-way valve means is provided for closing holes or openings and consists of an annular resilient member formed of a suitable material such as rubber. The resilient member has its inner margin seated in an annular recess provided in a part. The outer annular margin of the resilient member is free so that it can act as a one-way flapper valve for normally occluding the holes or openings and so that gases can only pass in one way through the openings.

U.S. Patent No. 4,534,343 discloses a metered dose inhaler for inhalation of asthmatic medication. The metered dose inhaler includes an air chamber. The bottom of the air chamber is open as a part of the molding process, and is closed by an elastomeric diaphragm having a pair of diametrical slits therein at right angles to one another. A single slit would suffice, but there is improved flexibility with two slits. A spider underlies the diaphragm, comprising an outer circular flange and at least two diametrical ribs arranged to underlie the slits.

U.S. Published Application No. 2005/0039746 discloses a ventilator circuit for use in administering medication to a patient. The ventilator circuit includes a chamber housing defining an interior space and having an input end, an output end, and a one-way inhalation valve positioned upstream of the interior space. The one-way inhalation valve is operative to permit a flow of medication into the interior space of the chamber housing. An inhalation conduit communicates with the output end of the chamber and is adapted to transmit the medication to the patient. An exhaust conduit is connected to the inhalation conduit and a one-way exhaust valve is located in the exhaust conduit. The one-way exhaust valve is adapted to prevent a backflow of gas from the exhaust conduit into the inhalation conduit.

U.S. Published Application Nos. 2004/0011358, 2004/0035413, 2005/0211253, 2005/0211245, and 2005/0325978 disclose methods, devices, and formulations for targeted endobronchial therapy. Aerosolized antibiotics are delivered into a ventilator circuit. The aerosol generator, e.g., nebulizer, may be placed in the lower part of a Y-piece.

U.S. Published Application Nos. 2005/0139211 and 2005/0217666 disclose devices, systems and methods applicable to endobronchial therapy.

There remains, however, a need for improved and more effective adapters for introducing aerosols into ventilator circuits.

### SUMMARY OF THE INVENTION

According to the present invention there is provided an adapter as claimed in the appended claims.

One or more embodiments of the present invention satisfies one or more of these needs. The present invention provides an adapter for use with apparatus to introduce aerosols, such as ventilator circuits and/or nebulizers. Also described are methods of treatment and/or prevention that utilize such valves, devices, adapters, systems, components, nebulizers, and/or ventilator circuits. Other features and advantages of the present invention will be set forth in the description of invention that follows, and will be apparent, in part, from the description or may be learned by practice of the invention.

Described is a valve. In one or more arrangements, the valve includes a support comprising at least one aperture. In other arrangements, the valve includes a support comprising a plurality of apertures. The support comprises a center and an outer edge. The valve also includes, in one or more arrangements, at least one flap, and in other arrangements, a plurality of flaps comprising a flap for each aperture. Each flap has an end connected proximal to the center of the support. Each flap is capable of moving between a closed position and an opened position.

In one or more arrangements, the valve includes a support comprising an aperture. The valve also includes at least one flap connected to the support. The valve further includes at least one protrusion on at least one member selected from the support and the flap, wherein the at least one protrusion on at least one member selected from the support and the flap has a surface that contacts the other of the support and the flap when the flap is in a closed position.

Another valve is also described. In one or more arrangements, the valve includes a support comprising an aperture. The valve also includes at least one flap connected to the support, the flap having a surface contacting the support when the flap is in a closed position. And the valve includes at least one channel in at least one member selected from the support and the flap. The at least one channel allows fluid flow through the valve when the flap is in the closed position.

According to the present invention, there is defined an adapter. In one or more embodiments, the adapter includes a housing forming a first channel and a second channel. The housing has a first end and a second end. The first channel comprises a first valve means, such as a one-way valve to allow flow in a first direction and impair flow in a second direction. The second channel comprises a second valve means, such as a one-way valve to allow flow in a third direction and impair flow in a fourth direction. The adapter also includes at least one of an aerosolization device and an aerosolization device port in the first channel positioned downstream, relative to the first direction, of the one-way valve. An air pressure drop between the first end and the second end of the adapter may be less than about 50 cm H2O at an air flow rate of 60 Umin, and may be less than about 40 or 30 or 20 or 10 cm H2O5 at an air flow rate of 60 Umin.

The adapter may further include a fluid accumulator in the second channel positioned upstream, relative to the third direction, of the second valve means, such as one-way valve.

The adapter optionally includes a sensor probe port, such as a temperature probe port, in the first channel positioned upstream, relative to the first direction, of the at least one of an aerosolization device and an aerosolization device port.

In yet another arrangement there is described a ventilator circuit. In one or more arrangements, the ventilator circuit comprises a ventilator, an exhalation line connected to the ventilator, and an inhalation line connected to the ventilator. The ventilator circuit also may include an adapter connected to the exhalation line and the inhalation line, the adapter comprising at least one of a nebulizer and a nebulizer port. A Y-piece is connected to the adapter. A tube selected from an endotracheal tube and a tracheostomy tube is connected to the Y-piece.

In other arrangements, a ventilator circuit comprises a ventilator, an exhalation line connected to the ventilator, and an inhalation line connected to the ventilator. The ventilator circuit may include an adapter connected to the exhalation line and the inhalation line, the adapter comprising at least one of a nebulizer and a nebulizer port. The expiration and inspiration lines may be arranged to be coaxial, or to be co-joined, such as a single divided line. In these embodiments, the Y-piece may be omitted, and the expiration and inspiration lines connected directly (or via a reducer, or adapter) to an endotracheal tube or a tracheostomy tube.

In other arrangements, the ventilator circuit comprises a ventilator, an exhalation line connected to the ventilator, an inhalation line connected to the ventilator, an adapter connected to the exhalation line and the inhalation line, the adapter comprising at least one of a nebulizer and a nebulizer port, and may further include a heat moisture exchanger (HME), such as an HME incorporated into an adapter.

In other arrangements, the ventilator is omitted, and an off vent device is provided, comprising a nebulizer, inhalation valve, exhalation valve and/or filter and, optionally, a holding chamber or reservoir. One or more off vent arrangements may be utilized with or without positive pressure assistance.

### BRIEF DESCRIPTION OF THE DRAWINGS

The present invention is further described in the description of invention that follows, in reference to the noted plurality of non-limiting drawings, wherein:
Fig. 1 is a side view of a first example of a valve which may be used in the present invention in a closed position.
Fig. 2 is a side view of the first example of the valve of Fig. 1 in an open position.
Fig. 3 is a cross-section of the first example of the valve of Fig. 1 in the closed position.
Fig. 4 is a cross-section of the first example of the valve of Fig. 1 in the opened position.
Fig. 5 is a cross-section of an outer edge of the first example of the valve of Fig. 1.
Fig. 6 is a top view of a support of the valve of the first example.
Fig. 7 is a top view of a flap of the valve of the first example.
Fig. 8 is a top view of a second example of a valve which may be used in the present invention, which example includes channels in a support.
Fig. 9 is a top view of a third example of a valve which may be used in the present invention, which example includes channels in flaps.
Figs. 10A-10B are top views of examples of flaps.
Figs. 11A-11B are cross-sections of examples of flaps.
Figs. 12A is a top view of another example of a flap. Fig. 12B is a cross-section of this example.
Fig. 13 is a cross-section of a further example of a valve which may be used in the present invention.
Fig. 14 is a top view of a support of the valve of Fig. 13.
Fig. 15 is a side view of another example of a valve which may be used in the present invention.
Fig. 16 is a top view of a support of the valve of Fig. 15.
Fig. 17 is a schematic view of an aerosolized pharmaceutical delivery system.
Fig. 18A is a perspective view of a first example of the adapter of the present invention.
Fig. 18B is a partial cross-section view of the first example of the adapter of the present invention.
Fig. 18C is another partial cross-section view of the first example of the adapter of the present invention.
Fig. 19 is a partial cross-section view of a second example of the adapter of the present invention.
Fig. 20A is a cross-section of an adapter of the present invention showing the fluid flow.
Fig. 20B is a flow profile diagram.
Fig. 21 is a perspective view of a portion of an adapter of the present invention, with an extension portion for receiving an aerosolization apparatus.
Fig. 22 is a cross-section of the embodiment of Fig. 21.
Fig. 23 is a perspective view of a portion of an adapter of the present invention, with a shorter extension portion.
Fig. 24 is a cross-section of the embodiment of Fig. 23.
Fig. 25 is a cross-section of an adapter of the present invention, with an extension portion having channels.
Fig. 26 is a cross-section of an extension portion of the present invention, with a cone-shaped sheath.
Fig. 27 is a cross-section of an adapter of the present invention, with an inverted extension portion.
Fig. 28 is a perspective of a nebulization system for use with an adapter of the present invention.
Fig. 29 is a perspective of the embodiment of Fig. 28, with a lid open.
Fig. 30 is a perspective of a nebulization system in which a container includes a vibrating mesh element and piezoelectric element.
Fig. 31 is a perspective showing a vial rupture element.
Fig. 32 is a perspective showing a nebulization system in which a piezoelectric element is contained within the structure of the vibrating mesh nebulizer in or near a vial receiver and transmits the vibration of proper frequency into vibrating mesh element through mechanical contact.
Fig. 33 is a perspective showing a nebulizer system in which the container does not require a vial receiver.
Fig. 34 is a perspective showing a nebulization system used with an adapter of the present invention.
Fig. 35 is a schematic showing an adapter of the present invention connected with tubing of a ventilator circuit.
Fig. 36 is a schematic showing an adapter of the present invention used with a heat/moisture exchange (HME) filter.

### DESCRIPTION OF THE INVENTION

Unless otherwise stated, a reference to a compound or component includes the compound or component by itself, as well as in combination with other compounds or components, such as mixtures of compounds.

As used herein, the singular forms "a," "an," and "the" include the plural reference unless the context clearly dictates otherwise.

Medicament, "active agent"or pharmaceutical may be used interchangeably, and individually or collectively comprise any drug, solution, compound or composition which induces a desired pharmacologic and/or physiologic effect, when administered appropriately to the target organism (human or animal).

Reference herein to "one embodiment", "one version" or "one aspect" shall include one or more such embodiments, versions or aspects, unless otherwise clear from the context.

Before further discussion, a definition of the following terms will aid in the understanding of the present invention.

"Creep" (or "delayed deformation") is deformation that is time-dependent and is exhibited by a material subjected to a sustained load. Creep may be measured by tensioning a test sample with a fixed load and periodically recording the elongation. Creep resistance, in this document, is measured by subjecting a sample at 23[deg.]C to a 55.2 bar (800 psi) load for 1000 hours.

As an overview, the present invention comprises an adapter. It is emphasized that each component described may be used independently of the combinations and/or assemblies described herein. Thus the valves are not limited to use with the adapters and ventilator circuits of the invention. Similarly, the adapters of the present invention are not limited to use with the valves and ventilator circuits described herein. Moreover, the ventilator circuits are not limited to use with the valves and adapters of the present invention.

One or more of the valves, adaptors, systems and circuits are configurable to administer aerosolized medicament to a patient both on-ventilator and off- ventilator. On-ventilator treatment methods comprise administering the nebulized aerosol through a ventilator circuit to the patient. Aerosol doses, containing an effective dose, such as about 1 to about 500 mg of a medicament, may be delivered through the ventilator circuit in a phasic or non-phasic manner. Off- ventilator treatment methods comprise taking the patient off the ventilator before administering the nebulized aerosol. Once the treatment session is completed the patient may be put back on the ventilator, or may breathe on his or her own without assistance. Off-Vent devices often are self-contained, for freely-breathing patients, and may comprise an aerosol generator (e.g. a nebulizer) and a mask, cannula, lipseal or mouthpiece to administer an aerosolized liquid or powder formulation, such as a medicament. Administration may be continuous, phasic (such as during inspiration), and/or intermittent (such as timed). Devices, especially off-vent devices, used to administer the aerosol formulations, such as medicaments, may comprise a reservoir or holding chamber to permit or allow continuous flow of aerosol. The valves, devices, adapters, systems and components may be used with positive pressure-type apparatus, or not.

The adapter of the present invention may be used in the treatment of a variety of ailments using a variety of aerosolizable medicaments. The ailments may comprise pulmonary ailments such as ventilator-associated pneumonia, hospital-acquired pneumonia, community-acquired pneumonia, cystic fibrosis, mycobacterial infection, bronchitis, staph infection, Staph infections including MRSA, fungal infections, viral infections, protozal infections, and acute exacerbation of Chronic Obstructive Pulmonary Disease, among others. The aerosolizable medicaments used to treat the ailments may include antibiotics, anti- oxidants, bronchodialators, corticosteroids, leukotrienes, protease inhibitors, and surfactants, among other medicaments.

In one or more arrangements of the valve, the valve includes a support comprising a plurality of apertures. The support comprises a center and an outer edge. The valve also includes a plurality of flaps comprising a flap for each aperture. Each flap has an end connected proximal to the center of the support. Each flap is capable of moving between a closed position and an opened position.

In other arrangements of the valve, the valve includes a support comprising an aperture. The valve also includes a flap connected to the support. The valve further includes at least one protrusion on at least one member selected from the support and the flap, wherein the at least one protrusion on at least one member selected from the support and the flap has a surface that contacts the other of the support and the flap when the flap is in a closed position.

In still other arrangements, the valve includes a support comprising an aperture. The valve also includes a flap connected to the support, the flap having a surface contacting the support when the flap is in a closed position. And the valve includes at least one channel in at least one member selected from the support and the flap. The at least one channel allows fluid flow through the valve when the flap is in the closed position.

In other embodiments of the adapter, the adapter includes a housing forming a first channel and a second channel. The housing has a first end and a second end. The first channel comprises a first one-way valve to allow flow in a first direction and impair flow in a second direction. The second channel comprises a second one-way valve to allow flow in a third direction and impair flow in a fourth direction. The adapter also includes at least one of an aerosolization device and an aerosolization device port in the first channel positioned downstream, relative to the first direction, of the one-way valve. An air pressure drop between the first end and the second end of the adapter is less than about 50 cm H2O at an air flow rate of about 60 Umin, and may be less than about 40 cm H2O, 30 cm H2O, 20 cm H2O, 10 cm H2O or less, at an air flow rate of about 60 Umin.

The adapter may further include a fluid accumulator in the second channel positioned upstream, relative to the third direction, of the second one-way valve.

The adapter may include a sensor probe port, such as a temperature probe port in the first channel positioned upstream, relative to the first direction, of the at least one of an aerosolization device and an aerosolization device port.

In one or more arrangements of the ventilator circuit, the ventilator circuit comprises a ventilator, an exhalation line connected to the ventilator, and an inhalation line connected to the ventilator. The ventilator circuit also includes an adapter connected to the exhalation line and the inhalation line, the adapter comprising at least one of a nebulizer and a nebulizer port. A Y-piece is connected to the adapter. A tube selected from an endotracheal tube and a tracheostomy tube is connected to the Y-piece.

In other arrangements of a ventilator circuit, the circuit may comprise a ventilator, an exhalation line connected to the ventilator, and an inhalation line connected to the ventilator. The ventilator circuit may include an adapter connected to the exhalation line and the inhalation line, the adapter comprising at least one of a nebulizer and a nebulizer port. The expiration and inspiration lines may be arranged to be coaxial, or to be co-joined, such as a single divided line. In these arrangements, the Y-piece may be omitted, and the expiration and inspiration lines connected directly (or via a reducer, or adapter) to an endotracheal tube or a tracheostomy tube.

In other arrangements, the ventilator circuit comprises a ventilator, an exhalation line connected to the ventilator, an inhalation line connected to the ventilator, an adapter connected to the exhalation line and the inhalation line, the adapter comprising at least one of a nebulizer and a nebulizer port, and may further include a heat moisture exchanger (HME), such as an HME incorporated into an adapter.

In other arrangements, the ventilator circuit comprises any of the foregoing components, except the ventilator is not considered a part of the circuit.

In other arrangements, the valves, devices, systems and circuits are used in an off- vent configuration, such as an apparatus comprising a nebulizer, inhalation valve, exhalation valve and/or filter and, optionally, a holding chamber or reservoir. In general, such devices or apparatus comprise an aerosol generator, such as a nebulizer, and some means for delivering the aerosol thus generated to the patient, especially a free-breathing patient. In some arrangements, such apparatus may further comprise a holding chamber to allow continuous aerosol generation while delivering in a non-phasic manner. Further examples of off- vent devices and systems are disclosed, for example, in commonly-owned United States Patent Application Publication No. 20050217666, filed March 24, 2005.

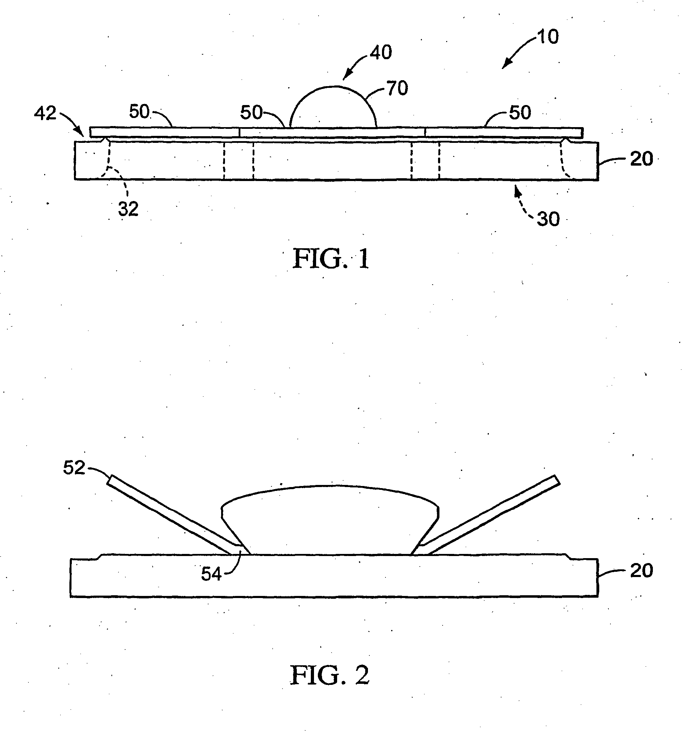

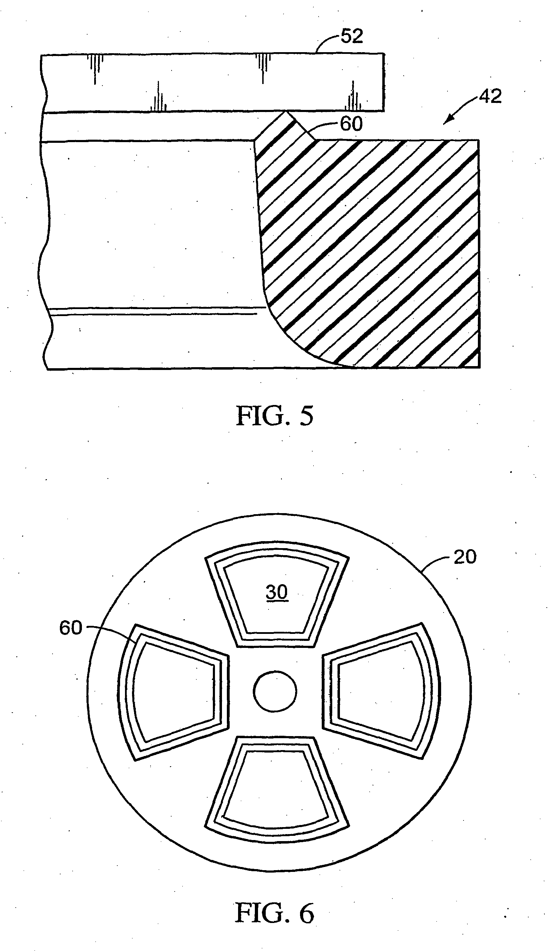

.An exemplary valve 10 is shown in Figs. 1-7. The valve 10 comprises a support 20. Although the support 20 is shown as being planar, the support 20 may take other forms. For example, the support may have a radius of curvature. The radius of curvature may range from about 0.1 cm to about 100 cm, such as about 1 cm to about 50 cm, or about 5 cm to about 10 cm.

The support 20 includes apertures 30. The valve 10 of Figs. 1-7 has four apertures 30, but the number of apertures 30 is not limited. For example, the valve 10 may have one or more apertures 30 such as one, two, three, four, five, six, or more apertures 30.

The apertures 30 are designed to allow fluid, such as liquids or gases (e.g., air), to pass through the support 20. The shape of the apertures is not particularly limited. Accordingly, the shape of a cross-section of the apertures may be circular, triangular, trapezoidal, square, star, oval, elliptical, irregular, or the like. The sides 32 of the apertures may be parallel to fluid flow or may be angled relative to the predominant fluid flow. Thus, the sides of the apertures may form an angle of less than about -90, -85, -80, -75, -70, -65, - 60, -55, -50, -45, -40, -35, -30, -25, -20, -15, -10, -5, 0, 5, 10, 15, 20, 25, 35, 40, 45, 50, 55, 60, 65, 70, 75, 80, 85, or 90 degrees relative to the predominant fluid flow. An aperture side angle of about 0[deg.] may reduce turbulence. An aperture side angle of other than about 0[deg.] may increase mixing of the fluid.

The valve 10 can be optimized to provide desired conditions and/or results. In some arrangements, the valve 10 is optimized to maximize fluid flow at a given flow resistance, or to minimize pressure drop at a given flow rate, or combinations thereof. For instance, the aperture(s) may form a high proportion of a cross-section of the valve that is in a plane normal to the predominant fluid flow. The aperture(s) may, e.g., comprise greater than about 10%, 20%, 30%, 40%, 50%, 60%, 70%, 80%, or 90%, of the valve cross-section. In other arrangements, the valve 10 is optimized to prevent or mitigate sticking, impediments to flow and/or mechanical failures. In other arrangements, the valve 10 is optimized to provide a desired or optimized sensitivity to pressure changes, such as to open at a desired low pressure differential. This can provide efficiencies in ventilator usage and/or medicament administration.

The valve 10 shown in the arrangement of Figs. 1-7 has a center area 40, including the geometric center, and an outer edge 42. The outer edge 42 is shaped to fit in a conduit (not shown). Alternatively, the outer edge may be part of the conduit, i.e., at least the valve support and the conduit are integral with each other. The outer edge 42 shown in the embodiment of Figs. 1-7 is circular. But the outer edge 42 is not limited to any particular shape and may be other shapes, such as elliptical, square, triangular, irregular, and the like.

The valve 10 also comprises a flap or flaps 50. The valve 10 shown in the arrangements of Figs. 1-7 includes four flaps 50, but the number of flaps is not limited. For example, the valve 10 may have one or more flaps 50 such as one, two, three, four, five, six, or more apertures. In general, each aperture 30 will have a corresponding flap 50, however one flap 50 may cover more than one aperture 30. Having a plurality of flaps provides redundancy. Thus, if one of the flaps should stick, one or more other flaps may still allow fluid to pass. In some arrangements, having a single flap may reduce the likelihood of sticking because gases would have a tendency to force open the single flap. In some arrangements, a single flap would also provide greater cross-sectional area for reduced flow resistance in high flow rate applications.

The flaps 50 shown in the arrangement of Figs. 1-7 have an end 52 connected to the center area 40 of the support 20. Alternatively, the flaps 50 may have an end connected to the support near the outer edge 42 of the support 20.

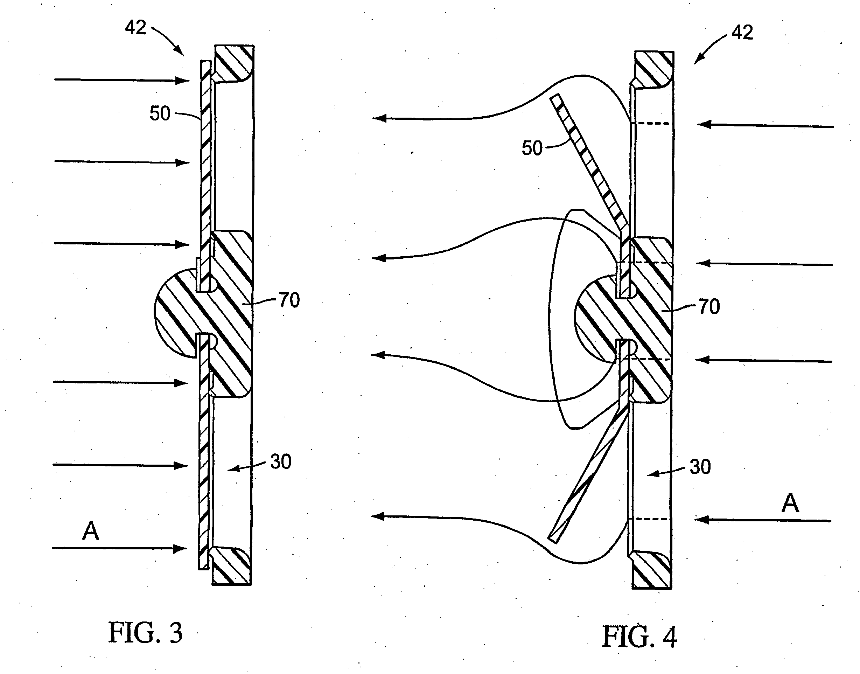

The flaps 50 may be in an opened or closed position. Thus, in operation, pivoting the flaps 50 in a first direction causes the flap to move to a closed position, shown in Fig. 1 and 3. Pivoting the flap in a second direction causes the flap to move to an opened position, shown in Fig. 2 and 4. Figs. 3 and 4 are cross-section views and show the effect of fluid flow (shown by arrows A) on the flaps 50.

The flaps 50 shown in the arrangement of Figs. 1-7 are biased in the closed position. Alternatively, the flaps 50 may be biased in the opened position. The flaps 50 shown in the arrangement of Figs. 1-7 are biased in the closed position by the shape memory of the flap material. In addition or alternatively, the flaps may be biased by a biasing means, such as a spring, secondary flapper, or prestressed material partially overlapping secondary flapper.

When the flaps 50 of the arrangement of Figs. 1-7 are in the closed position, the flaps 50 are resting on the support 20 and are under minimal or no compressive stress or load?. The minimal stress reduces the chance that the flaps 50 will stick to the support 20. Alternatively, the flaps 50 may be preloaded or biased in the closed position. The preload or bias may reduce leakage through the valve 10, such as in a back pressure scenario. The preload or bias may be achieved by memory materials, springs, and the like.

Thus, each flap 50 shown in the arrangement of Figs. 1 -7 comprises a moving end 52 opposite to the connected end 54. The moving end 52 typically contacts the outer edge 42 of the support 20 in the closed position. The outer edge 42 of the support 20 optionally comprises a protrusion 60 that contacts the flaps 50. Optionally, the protrusion(s) may form part of the flap(s) and contact the support when the valve is closed (not shown).

The protrusion 60 often functions as a seat to minimize the contact area between the support 20 and the flap 50. The minimized contact area may reduce the chances that the flap 50 will stick to the support 20.

The protrusions 60 of the arrangement shown in Figs. 1-7 are triangular and form a contiguous border around apertures 30. Thus, the protrusion forms a knife-edge. Shapes other than triangular may be used to form a contiguous border around the apertures 30. For example, the cross-section of the protrusions may be rectangular, half-circular, ellipsoidal, conic, or the like.

In some arrangements, the contact area of the contiguous border may be less than about 1 cm², such as less than about 0.5 cm², or less than about 0.1 cm², per 1 cm of border length. Contiguous borders may minimize leakage through valve 10.

In some arrangements, the contact area may be non-contiguous. For instance, the protrusions 60 may comprise regular or irregular needles, triangles, rectangles, cones, half cylinders, spheres, semi-spheres, pyramids, shark-fin shaped, crescents, sections thereof, or other shapes separated by gaps, or the like. Although the non-contiguous contact area may result in some leakage, such protrusions may reduce sticking. In this regard, the protrusions would reduce contact area and would self-clean because of surface tension. In certain applications, reducing the likelihood of sticking may be more important than reducing leakage. For instance, the adapters for adding an aerosol into a ventilator circuit, as discussed in more detail below, can in some cases tolerate leakages up to about 10 Umin, such as up to about 10 Umin, up to about 0.5 Umin, or up to about 0.1 L/min. If one or more flaps of a valve in one of these adapters sticks, the flow profile may become irregular.

In some applications, a slight leak through the valve may be desired. For instance, the adapters for adding an aerosol into a ventilator circuit, as discussed in more detail below, may advantageously include some leakage. In this regard, some leakage may improve the patient to ventilator interface.

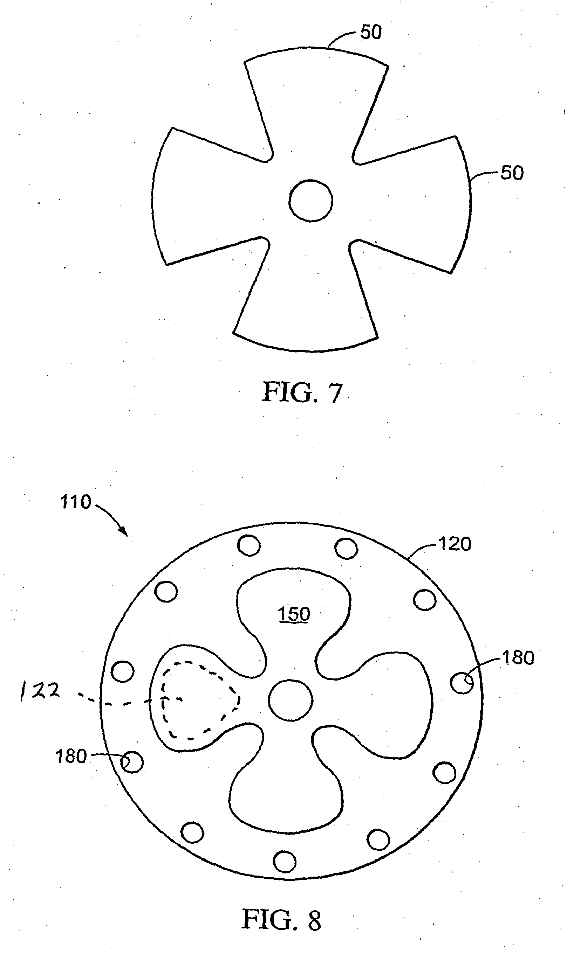

Thus, in one version shown in Fig. 8, the valve 110 includes a support 120 comprising apertures 122 (shown in phantom). Flaps 150 are connected to the support 120. The flaps 150 have a surface contacting the support 120 when the flaps 150 are in a closed position. The support 120 may include channels 180 that allow fluid flow through the valve 110 when the flaps 150 are in the closed position.

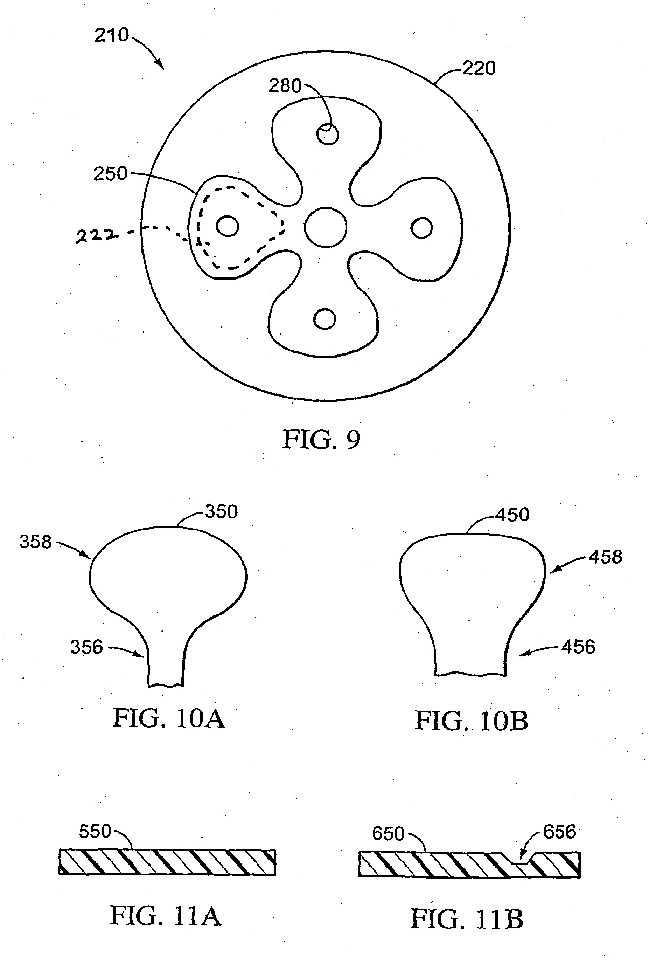

In another version shown in Fig. 9, the valve 210 includes a support 220 comprising apertures 222 (shown in phantom). Flaps 250 are connected to the support 220. The flaps 250 have a surface contacting the support 220 when the flaps 250 are in a closed position. The support 220 may include channels 280 that allow fluid flow through the valve 210 when the flaps 250 are in the closed position.

In the versions shown in Figs. 8 and 9, by varying the relative size and number of channels 180 and/or 280, a skilled artisan may vary the ratio of fluid flow through the valve when the flap is in an opened position to the fluid flow through the valve when the flap is in the closed position. For instance, the fluid flow in the opened position may be at least about 2 times, at least about 10 times, at least about 100 times, at least about 1000, at least about 10,000 times, or at least about 100,000 times greater than fluid flow in the closed position.

The amount of contact area per flap depends on factors such as the size of the flap. In some arrangements, the amount of contact area per flap include, but are not limited to, less than about 1 cm², less than about 0.8 cm², less than about 0.5 cm², less than about 0.1 cm², and less than about 0.01 cm². In some arrangements, the percentage of surface area of the surface of the flap(s) in contact with the protrusion(s) often ranges from about 0.1 % to about 50%, such as about 0.5% to about 25%, about 1% to about 10%, and about 1% to about 5%. The percentage of surface area of the surface of the flap(s) in contact with the protrusion(s) is often less than about 5%, such as less than about 2.5%, less than about 1%, less than about 0.5%, less than about 0.1%, or less than 0.01%.

The flaps 50 in the arrangement of Figs. 1-7 are integral with each other, as shown in Fig. 7. The flaps 50 form a four-lobed structure similar to a four-leaf clover. Making the plurality of flaps integral with each other can reduce manufacturing costs. Alternatively, the plurality of flaps 50 may be distinct parts. Making the plurality of flaps 50 distinct parts may be desirable to prevent stresses in one flap from affecting the performance of another flap.

The support 20 and plurality of flaps 50 in the arrangement of Figs. 1-7 are distinct parts. Making the support 20 and plurality of flaps 50 distinct parts allows optimization of the materials forming these parts. Alternatively, the support 20 and plurality of flaps 50 may be integral with each other. Making the plurality of flaps integral with each other can reduce manufacturing costs.

When the support 20 and plurality of flaps 50 are distinct parts, the flaps 50 can be mounted on the support using various techniques. As shown in Fig. 1, a center post 70 holds the flaps 50. Examples of mounting techniques are those known in the art and comprise adhesive fastening, mechanical fastening and material joining, such as snap-fitting, adhesive bonding, co-melting ultrasonic welding, RF welding, spin welding, clamping, hinging (as discussed below), and the like.

The material forming the plurality of flaps may be rigid or may be flexible. The flap material may be selected to allow the valve to open at low pressure drops. For instance, the flexible flap materials may have a Shore A hardness ranging from about 20 to about 90, such as about 30 to about 80, about 40 to about 70, and about 50 to about 60. Rigid materials have a Shore A hardness greater than 90. For instance, the rigid material may have a stiffness of at least 50 Rockwell B, such as 100 Rockwell B.

When the flap material is flexible, it may comprise a material with good shape memory or creep resistance or both. For instance, the creep resistance may be, e.g., less than about 4% elongation, such as less than about 3%, less than about 1%, less than about 0.5%, or less than about 0.2%, under a load of 55.2 bar (800 psi) at 23°C after 1000 hours.

When the flap material is rigid, the flap may be connected to the support by a hinge or hinge means. Examples of hingemeans include, but are not limited to, pin joints and living hinges.

The flaps may assume various shapes. For instance, Fig. 10A is a top view of a flap 350 having a narrow neck 356 and a head 358. If the flap 350 is formed of an elastomer, the narrow neck increases the likelihood that the flap will bend at the neck 356. In contrast, Fig. 10B is a top view of a flap 450 having a broad neck 456 and a head 458. If the flap 450 is formed of an elastomer, the broad neck 456 increases the likelihood that the flap 450 will bend over the length of the flap. The ratio of the width of the neck to the width of the head typically ranges from about 1 :20 to about 2:1, such as about 1 : 10 to about 1:1, about 1:5 to about 1:2.

The flaps may also have a neck formed in their cross-section. For instance, Fig. 1 IA is a cross-section view of a flap 550 that has a rectangular cross-section. If the flap 550 is formed of an elastomer, the flap 550 would tend to bend over its entire length. Fig. 1 IB is a cross-section view of a flap 650 that has a neck 656 formed in its cross-section. If the flap 650 is formed of an elastomer, the flap 650 would tend to bend or pivot at its neck 656. The ratio of the width at the neck 656 to the width of the flap 650 at other positions often ranges from about 1:10 to about 9:10, such as from about 1:8 to about 4:5, about 1:6 to about 7:10, and about 1:4 to about 3:5.

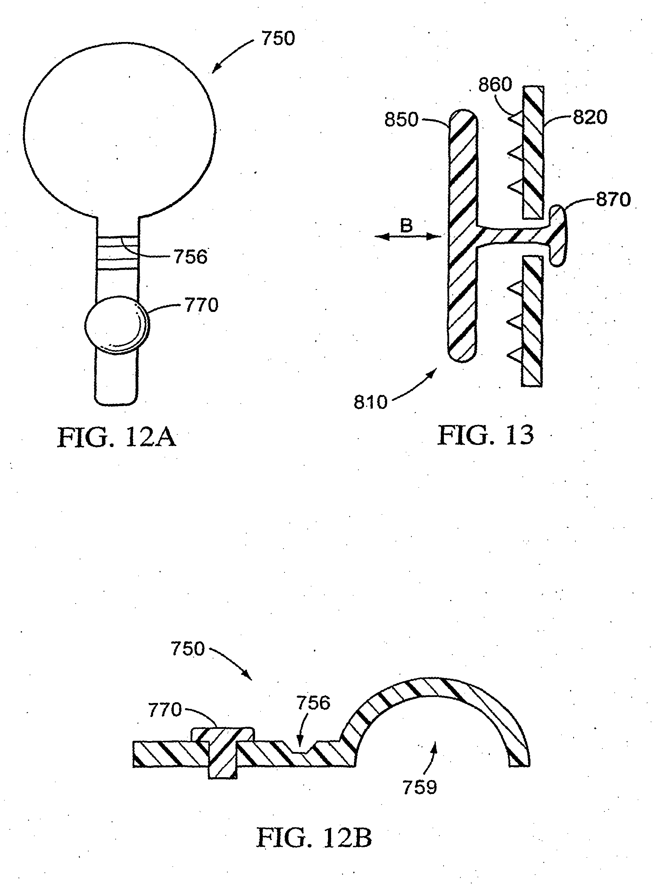

Fig. 12A is a top view of another version of the flap 750. The flap 750 includes a center post 770 for mounting the flap on a support. In some arrangements, the flap 750 also includes a neck 756 to facilitate pivoting of the flap 750. In some arrangements, the flap 750 includes a cup portion 759, as shown in Fig. 12B, which is a cross-section view.

The flap material and the support material may be the same or different. Examples of suitable flap and support materials include, but are not limited to, elastomers, polymers, metals, ceramics, and composites. Examples of polymers include, but are not limited to, polyurethane, fluoropolymers (e.g., polytetrafluoroethylene), nylons, silicone, such as silicone rubbers (e.g., available from Dow Chemical and GE), for instance two-part injection molded silicone rubber, etc., ethylene propylene diene monomer (EPDM), and Santoprene™ thermoplastic elastomers (available from ExxonMobil Chemical). Examples of composites include, but are not limited to, reinforced materials and laminates. The reinforced materials may include, e.g., particle-reinforced materials, fiber-reinforced materials, and silicone with a woven reinforcement. The laminates may include, e.g., parylene coated silicone, polytetrafluoroethylene coated polymer, polyimide adhered to polymer, reinforcement layer laminated on silicone, and low durometer polymer on high durometer polymer (e.g., 30 Shore A silicone on 90 Shore A silicone).

The valve can be designed such that fluid flow through the valve is desirably laminar or turbulent under appropriate conditions. To obtain predominantly laminar flow, the valve may be designed with few obstructions. Laminar flow may help minimize condensation. To obtain predominantly turbulent flow, the valve may be designed with obstructions or unbalanced flow path openings. Turbulent flow may increase mixing.

The valve 10 can be designed to minimize the likelihood of inversion. Inversion from a cough or other high back pressure can be irreversible such that the valve may stick in one configuration. Inversion can create serious safety issues, such as when the valve is used in a ventilator circuit. Valve inversion may also reduce the emitted dose of systems involving adapters for aerosolization devices. Inversion can be controlled, e.g., by overlapping the flaps 50 with the protrusion(s) 60. The valve 10 does not invert when air pressure changes in an amount of less than about 0.198 bar (2 psi), such as less than about 0.069 bar (1 psi) or less than 0.0945 bar (0.5 psi), in less than 0.5 second.

The valve can be designed to open at various pressures. For example, the flap may open when the pressure reaches a level ranging from about 0.05 cm H₂O to about 150 cm H₂O, such as about 0.1 cm H₂O to about 100 cm H₂O, about 0.5 cm H₂O to about 50 cm H₂O, about 1 cm H₂O to about 10 cm H₂O, or about 2 cm H₂O to about 5 cm H₂O.

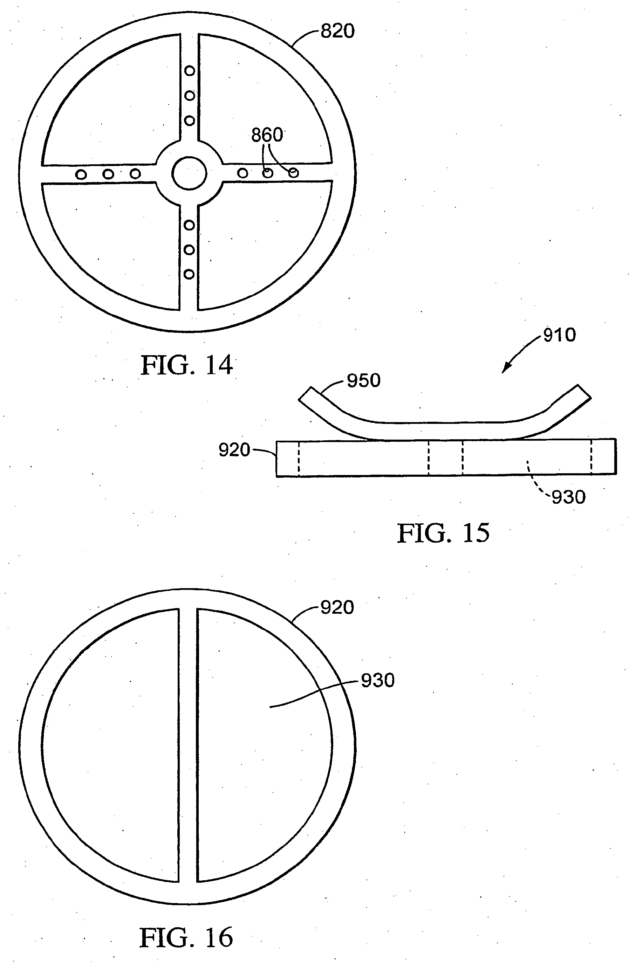

Another arrangement of the valve 810 is shown in Figs. 13 and 14. In this example, a flap 850 includes a center post 870 that is disposed in a support 820 to allow movement in the directions shown by arrow B. Fig. 13 shows the valve 810 in an opened position. When the valve 810 is in a closed position, the flap 850 contacts protrusions 860. As shown in Fig. 14, the protrusions 860 are spaced from one another. To avoid sticking, the spacing should be sufficient to prevent the space between the protrusions 860 from filling with liquid. For example, the protrusions may be spaced from one another by distance of less than about 1.5 cm, such as less than about 1 cm, less than about 0.2 cm, such as less than about 0.1 cm, less than about 0.05 cm, or less than about 0.005 cm.

Still another version of the valve 910 is shown in Figs. 15 and 16. Fig. 15 shows the valve 910 in an opened position. The support 920 holds a flap 950 that comprises a circular disc of flexible material. Fig. 16 is a top view of the support 920 without the flap 950. Although the support 920 of this example is shown with two apertures 930, the number of apertures is not particularly limited. Accordingly, the valve 910 may include two, three, four, five, six, or more apertures.

The valve may be used as a one-way valve to control fluid flow, especially gas flow, in a variety of circumstances. Uses for one-way valves are known. For example, the valve may be used in chemical processing, scuba gear, gas masks, ventilators (e.g., mechanical ventilators, manual ventilators), adapters for nebulizers, or the like.

Various techniques may be used to mount the valves in conduits. Examples of mounting techniques include, but are not limited to, snap-fitting, press fitting, threading, keying, adhesive bonding, ultrasonic welding, ultrasonic welding, RP welding, spin welding, clamping, and the like.

As noted above, the valves may be used in ventilator circuits and adapters for nebulizers. The ventilator circuits and adapters for nebulizers may take various forms. For example, the valve may be used in the adapters shown in commonly-owned U.S. Patent Publication No. 20050139211, filed November 17, 2004.

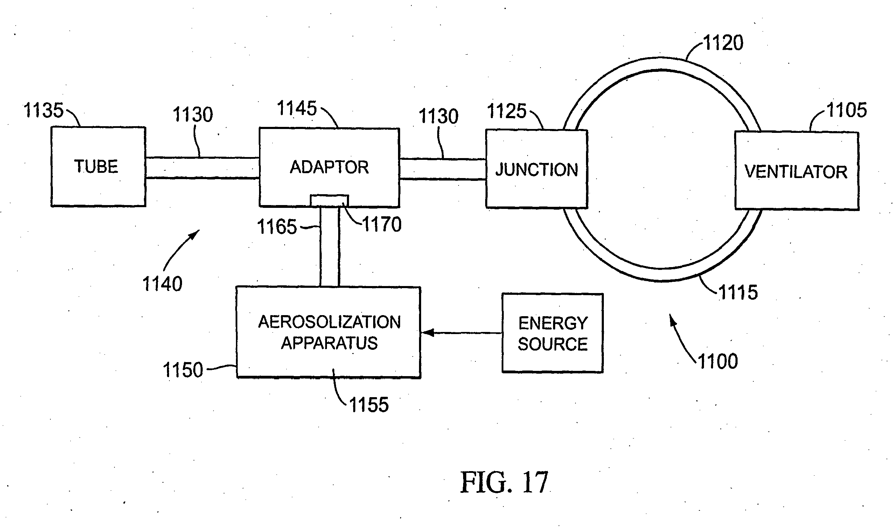

Thus, the valve may be used in an aerosolized pharmaceutical formulation delivery system 1100 as shown in Fig. 17. The aerosolized pharmaceutical formulation delivery system 1100 delivers an aerosolized pharmaceutical formulation to a portion of a user's respiratory tract, such as the user's lungs. In one or more arrangements, the aerosolized pharmaceutical formulation delivery system 1100 is useful in delivering the aerosolized pharmaceutical formulation to a patient whose breathing is being assisted by a ventilator 1105 but may also be configured to be used to deliver a pharmaceutical formulation to a non- ventilated patient, as discussed below. The ventilator circuit 1110 is shown diagrammatically in Fig. 17. Extending from the ventilator 1105 is an inhalation line 1115 and an exhalation line 1120. The inhalation line 1115 and the exhalation line 1120 are both composed of tubing having an airflow lumen extending therethrough. The inhalation line 1115 and the exhalation line 1120 meet at a junction 1125 remote from the ventilator 1105. At the junction 1125 the lumen of the inhalation line 1115 is in communication with the lumen from the exhalation line 1120, and both lumens are in communication with a patient line 1130. The patient line 1130 comprises a lumen that extends to the lumen of an endotracheal or tracheostomy tube 1135, which is inserted into a patient. The tube 1135 has an opposite end that may extend into or near the lungs of the user. Accordingly, oxygenated air is introduced into the inhalation line 1115 by the ventilator 1105. The oxygenated air passes through the lumen of the inhalation line 1115, into the patient line 1130, through the lumen of the tube 1135, and into the lungs of the patient. The patient then exhales, either naturally or by applying negative pressure from the ventilator, and the exhaled air passes through the tube 1135, through the patient line 1130, and through the exhalation line 1120 to the ventilator 1105. The cycle is continuously repeated to assist the patient's breathing or to entirely control the breathing of the patient.

The aerosolized pharmaceutical formulation delivery system 1100 further comprises an aerosol introduction means, such as a system or mechanism 1140. The aerosol introduction mechanism 1140 comprises an adapter 1145 that introduces aerosolized pharmaceutical formulation into the ventilator circuit 1110 at a position between the junction 1125 and the lungs of the patient. For example, the aerosol introducer may introduce the aerosolized pharmaceutical into the patient line 1130, as shown in Fig. 17, or may introduce the aerosolized pharmaceutical formulation within or near tube 1135. The aerosol that is introduced by the adapter 1145 is generated by an aerosolization apparatus 1150, which comprises a reservoir for containing a pharmaceutical formulation. Aerosolization energy is supplied to the aerosolization device by an energy source 1160 to generate the aerosolized pharmaceutical formulation. The aerosolized pharmaceutical formulation passes through a passage 1165 to the adapter 1145 where it may be introduced into the ventilator circuit 1110.

The aerosolization apparatus 1150 may be, for example, a jet nebulizer where the energy source is compressed air, a vibrating mesh nebulizer where the energy source is mechanical, such as wave, energy, an ultrasonic nebulizer where the energy source is acoustic wave energy, a metered dose inhaler where the energy source is a propellant, such as a composition that boils under preselected, such as ambient conditions, or a dry powder inhaler where the energy source is compressed or flowing air or is a vibrating membrane or the like.

Liquid formulations can be atomized by any of a variety of procedures. For example, the liquid can be sprayed through a two-fluid nozzle, a pressure nozzle, or a spinning disc, or atomized with an ultrasonic nebulizer or a vibrating orifice aerosol generator (VOAG). In one or more embodiments, a liquid formulation is atomized with a pressure nozzle, such as a BD AccuSpray nozzle. The aerosolization apparatus 1150 may be based on condensation aerosolization, an impinging jet technique, electrospray techniques, thermal vaporizing, or a Peltier device.

Jet nebulizers involve use of air pressure to break a liquid solution into aerosol droplets. In one or more embodiments, a jet nebulizer (e.g., Aerojet, AeroEclipse, Pari L. C., the Parijet, Whisper Jet, Microneb®, Sidestream®, Acorn 11®, Cirrus and Upmost®) generates droplets as a mist by shattering a liquid stream with fast moving air supplied by tubing from an air pump. Droplets that are produced by this method typically have a diameter of about 2-5 µm.

An ultrasonic nebulizer that uses a piezoelectric transducer to transform electrical current into mechanical oscillations may be used to produce aerosol droplets. Examples of ultrasonic nebulizers include, but are not limited to, the Siemens 345 UltraSonic Nebulizer(TM) and ones commercially available from, for example, Omron Heathcare, Inc. and DeVilbiss Health Care, Inc. See, e.g., EP 1 066 850. The resulting droplets typically have an MMAD in the range of about 1 to about 5 microns.

Vibrating porous plate nebulizers work by using a sonic vacuum produced by a rapidly vibrating porous plate to extrude a solvent droplet through a porous plate. See, e.g., U.S. Patent Nos. 5,758,637; 5,938,117; 6,014,970; 6,085,740; and 6,205,999.

For example the aerosol generator is the commercially available Aerogen (Aerogen, Inc. Mountain View, CA) aerosol generator which comprises a vibrational element and dome-shaped aperture plate with tapered holes. When the plate vibrates several thousand times per second, such as about 100 k/s to about 150 k/s, a micro-pumping action causes liquid to be drawn through the tapered holes, creating a low- velocity aerosol with a precisely defined range of droplet sizes. The Aerogen aerosol generator does not require propellant.

In the Aerogen Aeroneb and Pari eFlow (Pari Respiratory Equipment, Germany), a piezoelectric oscillator is placed circumferentially around the vibrating mesh and vibrations shake precisely sized droplets of the nebulizer content through the membrane, to form a respirable mist of medication on the other side. In another vibrating mesh nebulizer, the Omron Micro-air (Omron, Japan), the piezoelectric oscillator is positioned proximal to the vibrating mesh instead of circumferentially around it, pushing rather than shaking droplets of droplets of nebulizer content through the pores in the membrane with a similar result.

In condensation aerosol generators, the aerosol is formed by pumping drug formulation through a small, electrically heated capillary. Upon exiting the capillary, the formulation is rapidly cooled by ambient air, and a gentle aerosol is produced that is relatively invariant to ambient conditions and the user inhalation rate. See, e.g., U.S. Patent No. 6,701,922 and WO 03/059413. The condensation aerosol generator may comprise one disclosed by Alexza Molecular Delivery Corporation. See, e.g., U.S. Published Application No. 2004/0096402.

Another apparatus for delivery of a metered quantity of a liquid pharmaceutical composition for inhalation is described for example in WO 91/14468 and WO 97/12687. The nebulizers described therein are known by the name Respimat®.

One or more electrosprays may be used to nebulize liquid formulations. The term electrostatic spray (also known as electrohydrodynamic spray or electrospray) refers to systems in which the dispersion of the liquid relies on its electric charging, so that nebulization and gas flow processes are relatively uncoupled. Examples of electrospray devices are disclosed in U.S. Patent Nos. 6,302,331; 6,583,408; and 6,803,565.

The aerosol generator may comprise a thermal vaporizing device. Such a device may be based on inkjet technology.

The aerosol generator may comprise a Peltier device. An example of such a device is disclosed in U.S. Published Application No. 2004/0262513.

The aerosol generator may comprise a vibrating orifice monodisperse aerosol generator (VOAG). This device is an example of one type of monodisperse aerosol generator.

The aerosol generator may comprise a thin film, high surface area boiler that relies on capillary force and phase transition. By inducing phase transition in a capillary environment, pressure is imparted onto the expanding gas, which is ejected. This technology has been disclosed by Vapore, Inc., and is known as Vapore-Jet CFV technology. See, e.g., U.S. Patent Nos. 5,692,095; 5,870,525; 6,162,046; 6,347,936; 6,585,509; and 6,634,864, and U.S. Application No. 10/691,067.

Examples of the adapter 1145 for introducing the aerosolized pharmaceutical formulation at a position between the junction 1125 and the lungs of the patient is described in WO 2004/071368 as well as U.S. Published Application Nos. 2004/0011358 and 2004/0035413. Other examples of the adapter 1145 are disclosed in U.S. Patent Publication No. 20050139211 (infra).

The introduction of the aerosolized pharmaceutical formulation at a position between the junction 1125 and the lungs of the patient is advantageous in many respects over systems where the aerosol is introduced into the inhalation line 1115 or within the ventilator 1105. For example, by introducing the aerosolized pharmaceutical formulation at a position between the junction 1125 and the lungs of the patient, the ventilator circuit volume from the point of introduction to the patient's lungs is substantially reduced. Accordingly, the aerosolized pharmaceutical formulation is more concentrated and is less diffused throughout the ventilator circuit 1110. In addition, if the formulation is added in the inhalation line 1115, much of the formulation is drawn into the exhalation line 1120, further limiting the efficiency of the administration. Because of this diffusion and reduced efficiency, the consistency of dosing is difficult to control in known systems. Also, the presence of high quantities of the aerosolized pharmaceutical formulation that are not administered to the lungs of the patient may be undesirable in that much of the aerosol may be introduced into the environment where it may be inhaled by healthcare workers or others.

While the introduction of the pharmaceutical formulation at a position between the junction 1125 and the lungs of the patient is advantageous over known systems, it has been discovered that, in some circumstances, much of the introduced aerosolized pharmaceutical formulation may still be drawn into the exhalation line 1120 prior to being administered to the patient. Therefore, the adapter 1145 of the invention has been designed to introduce the aerosolized pharmaceutical formulation in an improved manner to increase the efficiency and/or the consistency of the dosing. Accordingly, the adapter 1145 introduces the aerosolized pharmaceutical formulation into the inhalation flow at a position between the junction 1125 and the lungs of the patient. In this way, the adapter 1145 serves to reduce the amount of aerosolized pharmaceutical formulation that is drawn into the exhalation line 1120 of the ventilator circuit 1120.

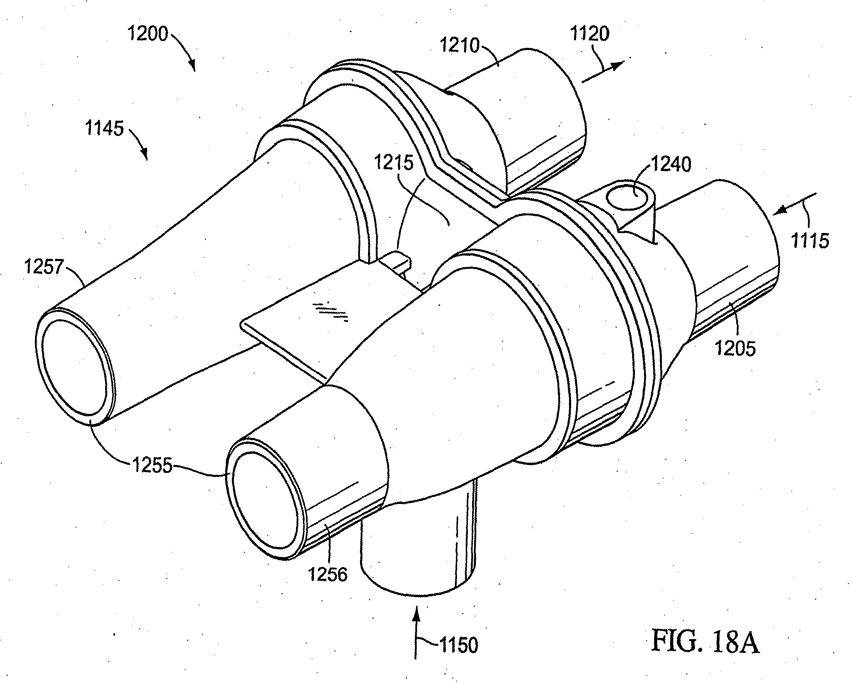

Figs. 18A-18C show a version of the adapter 1145 that also performs the function of Y-piece junction 1125. The aerosol introducer 1145 of Figs. 18A-18C comprises an H-shaped body 1200. At a first end of the H-shaped body 1200, a first connector 1205 and a second connector 1210 are adapted to be connectable to an inhalation line 1115 and an exhalation line 1120 of a ventilator circuit 1110, respectively. Within the H-shaped body 1200, a cross channel 1215 provides a lumen so that air may flow from the first connector 1205 to the second connector 1210. As such, the connectors 1205, 1210 and the cross channel 1215 serve as the junction 1125 of the inhalation line 1115 and the exhalation line 1120 in a manner similar to that of a conventional Y-piece.

A wall 1255 in this version is in the form of two tubes 1256, 1257 that define the first channel 1265 and second channel 1260, respectively. The first channel 1265 includes an extension portion 1285 that is in communication with the aerosolization apparatus 1150 and is able to receive aerosolized pharmaceutical formulation.

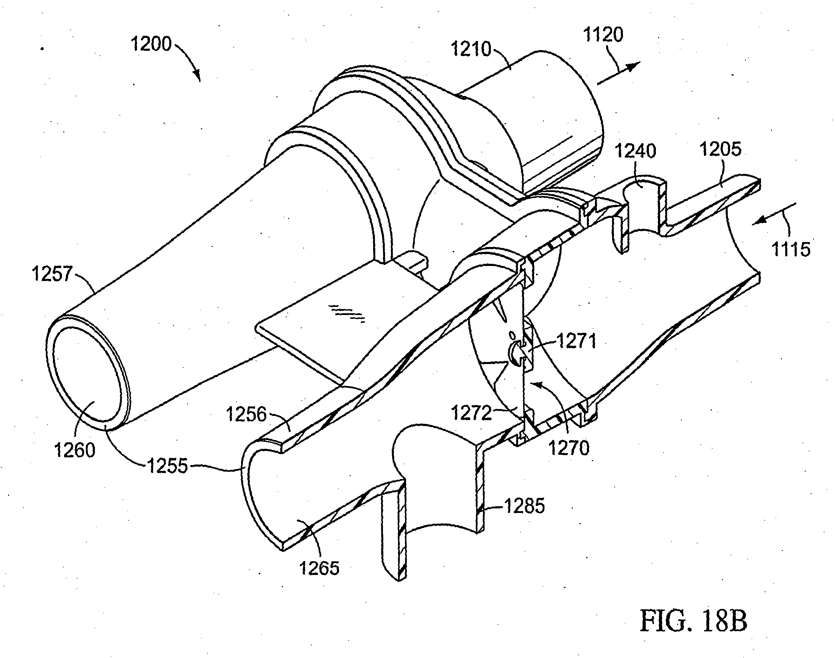

As best shown in Fig. 18B, within the first channel 1265 and at a position downstream (relative to the inhalation direction) of the cross channel 1215, a one-way inhalation valve 1270, as discussed above, is provided. In this version, the one-way inhalation valve 1270 comprises a support 1271 that holds flaps 1272. The one-way inhalation valve 1270 opens during inhalation and closes during exhalation.

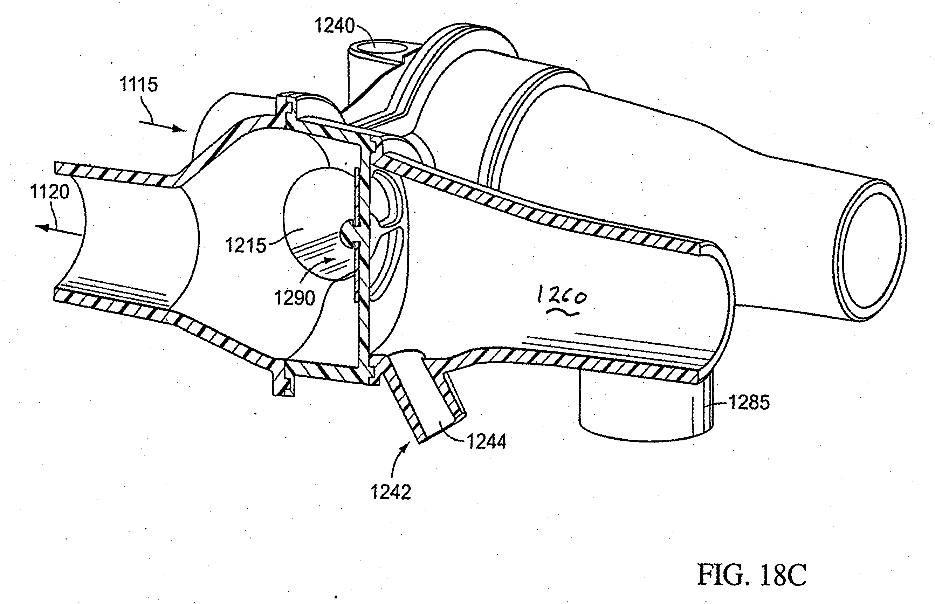

As best shown in Fig. 18C, within the second channel 1260 and at a position upstream (relative to the exhalation direction) of the cross channel 1215, a one-way exhalation valve 1290, as discussed above, is provided. The one-way exhalation valve 1290 opens during exhalation and closes during inhalation.

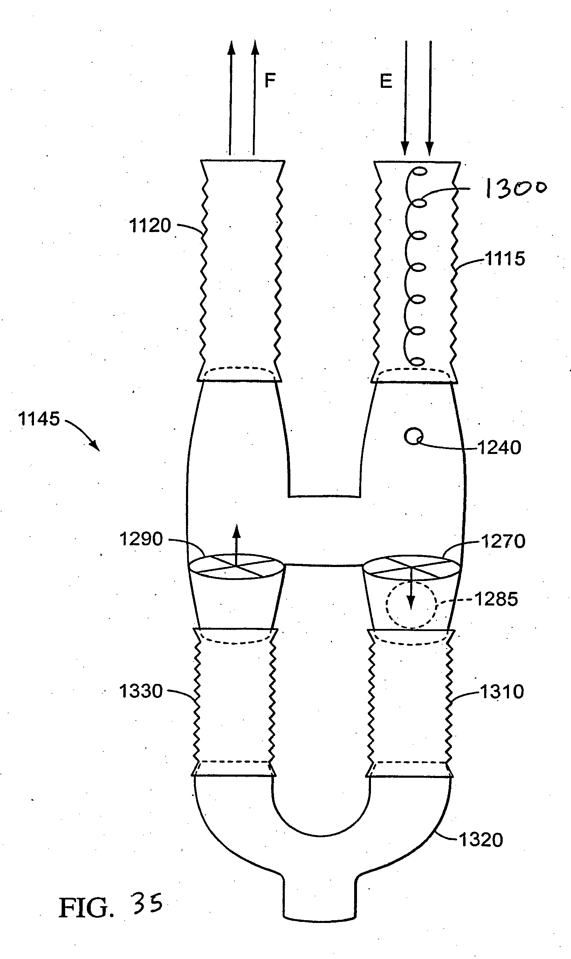

The adapter 1145 also includes a sensor probe port 1240 for use with a sensor probe, such as a temperature probe for a heated wire humidifier (see Fig. 35, discussed below). Examples of suitable temperature probes include, but are not limited to, resistance temperature detectors, thermistors, thermocouples, Fisher-Paykel 561 temperature probes, Hudson RCI temperature probes, and the like. Other sensors may comprise pressure sensors, humidity (or moisture) sensors, air flow sensors, or combinations thereof.

Measuring the temperature of the inhalation gas at this point is advantageous because it reflects the temperature of the inhalation gas before the aerosolization apparatus 1150 introduces gas. If the temperature of inhalation gas is measured at a point after the aerosolization apparatus 1150 introduces gas, the inhalation gas may be overheated before reaching the adapter 1145 or control of the ventilated gas heating function may be compromised.

The adapter 1145 may include a fluid accumulator 1242 in the second channel 1260 positioned upstream, relative to the exhalation direction, of the one-way exhalation valve 1290. The fluid accumulator is arranged to prevent fluid, e.g., condensation and/or mucus, from affecting the one-way exhalation valve 1290. In this regard, the one-way exhalation valve 1290 may be elevated relative to where fluid accumulates. For instance, the bottom of the second channel 1260 may be at least about 2 cm, such as at least about 1 cm, or at least about 0.5 cm, below the bottom of the one-way exhalation valve 1290.

The fluid accumulator 1242 shown in Figs. 18A-18C comprises a port 1244. The port may include a valve (not shown). Examples of the valve include, but are not limited to, stop-cocks, sphincter valves, injection sites, removable caps, and the like. A needless syringe may be used to suction out fluids.

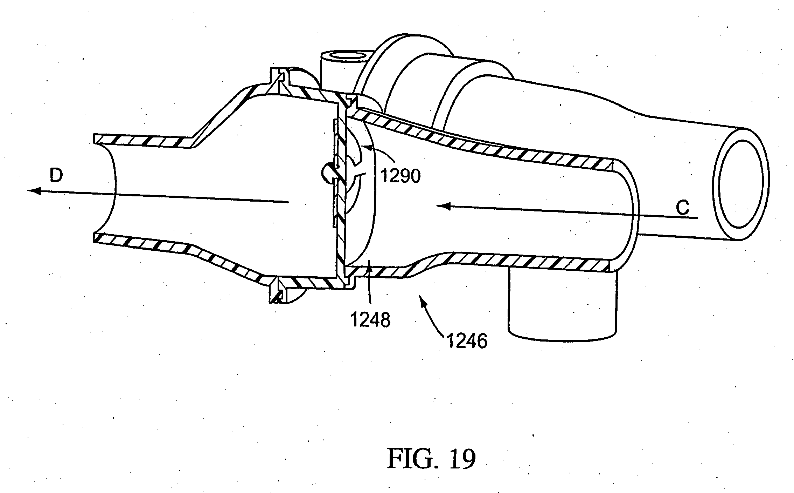

Alternatively, as shown in Fig. 19, the fluid accumulator 1242 may omit the port 1244 and comprise a reservoir 1246 formed in the second channel 1260. Thus, fluid 1248 may accumulate in the reservoir 1246. The reservoir may typically hold at least about 20 ml, such as at least about 10 ml, or at least about 5 ml, of fluid before the one-way exhalation valve 1290 contacts the fluid. In Fig. 19, arrow C depicts air returning from a patient, and arrow D depicts air returning to a ventilator.

The adapters of the present invention when used in a ventilator circuit are often able to reproducibly and efficiently deliver pharmaceutical formulation. For instance, the present invention is typically able to reproduce the delivered dose within about ± 10%, ± 8%, ± 6%, ± 4%, ± 2%, or ± 1%, of the total nominal dose. The present invention is often able to achieve a delivered efficiency of at least about 30%, such as at least about 40%, at least about 50%, at least about 60%, at least about 70%, at least about 80%, or at least about 90%.

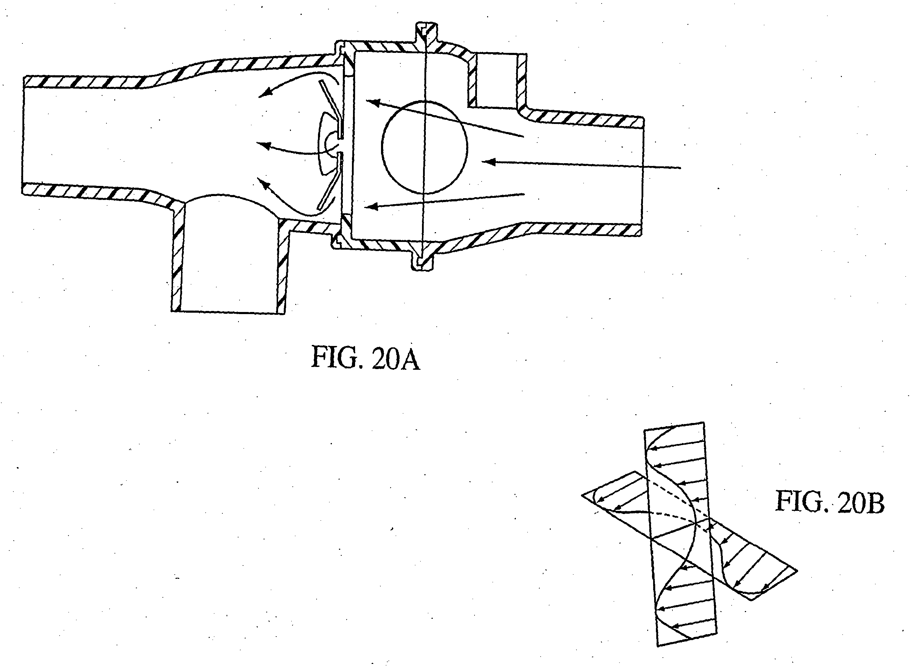

Many versions of the present invention are able to achieve this reproducibility and efficiency, in part, because of the flow profile of air passing through the adapter. Figs. 20A and 20B show a flow profile of the air. Fig 20B is an idealized, schematic representation of a cross section of air flow through the valve of Fig 20A (a four-flap coverleaf) and shows air flow around the valve, within the adapter channel. As illustrated by Figs 20, since high velocity air passes close to the surface of the adapter, the adapter is self-cleaning.

The adapter of the present invention typically advantageously has minimal impact on the patient to ventilator interface. The minimal impact allows the ventilator to react more efficiently to the patient. The adapter and valves are arranged so that at an air flow rate of 60 L/min, the pressure drop between the first end and the second end of the adapter is often less than about 50 cm H₂O, such as less than about 30 cm H₂O, less than about 5 cm H₂O, less than about 4 cm H₂O, less than about 3 cm H₂O, less than about 2 H₂O, or less than about 1 cm H₂O, and may range from about 0.05 cm H₂O to about 10 cm H₂O, about 1 cm H₂O to about 5 cm H₂O, or about 2 cm H₂O to about 4 cm H₂O. At an air flow rate of 30 Umin, the pressure drop between the first end and the second end of the adapter is typically ranges from about 1 cm H₂O to about 2 cm H₂O.

The adapter may be made of a transparent, translucent, or opaque material. Using a transparent material is advantageous because the user can visually inspect the functioning of the adapter. Examples of materials for the adapter include, but are not limited, to polymers, such as polypropylene, SAN (styrene acrylonitrile copolymer), ABS (acrylonitrile-butadiene-styrene), polycarbonate, acrylic polysulfone, K-resin® styrenebutadiene-copolymer (available from Chevron Phillips Chemical), polyethylene, PVC (polyvinyl chloride), polystyrene, and the like.

The aerosolization apparatus 1150 may be of any type that is capable of producing respirable particles or droplets. For example, the pharmaceutical formulation may be in dry powder form, as described in WO 99/16419; U.S. Patent No. 6,051,256; or U.S. Patent No. 6,503,483. In such cases, the aerosolization apparatus 1150 may comprise an active dry powder aerosolization apparatus, such as an aerosolization apparatus described in U.S. Patent Nos. 5,485,135; 5,740,794; or 6,257,233, or a passive dry powder aerosolization apparatus, such as an aerosolization apparatus described in U.S. Patent Nos. 4,069,819 or 4,995,385. Alternatively, the pharmaceutical formulation may comprise dissolved in or suspended in a liquid propellant, as described in U.S. Patent Nos. 5,225,183; 5,681,545; 5,683,677; 5,474,759; 5,508,023; 6,309,623; or 5,655,520. In such cases, the aerosolization apparatus 1150 may comprise a metered dose inhaler (MDI). Alternatively, the pharmaceutical formulation may be in a liquid form and may be aerosolized using a nebulizer as described in WO 2004/071368 as well as U.S. Published Application Nos. 2004/0011358 and 2004/0035413. Other examples of nebulizers include, but are not limited to, the Aeroneb®Go or Aeroneb®Pro available from Aerogen, Inc. of Mountain View, CA; the PARI eFlow and other PARI nebulizers available from PARI Respiratory Equipment, Inc. of Midlothian, VA; the Lumiscope® Nebulizer 6600 or 6610 available from Lumiscope Company, Inc. of East Brunswick, NJ; and the Omron NE-U22 available from Omron Healthcare, Inc. of Kyoto, Japan.

It has been found that an adapter with a nebulizer that forms droplets without the use of compressed gas, such as the Aeroneb® Pro and the PARI eFlow, provides unexpected improvement in dosing efficiency and consistency. By generating fine droplets by using a vibrating perforated or imperforated membrane, rather than by introducing compressed air, the aerosolized pharmaceutical formulation can be introduced into the ventilator circuit 1110 without substantially affecting the flow characteristics within the circuit and without requiring a substantial re-selection of the ventilator settings. In addition, the generated droplets when using a nebulizer of this type are introduced at a low velocity, thereby decreasing the likelihood of the droplets being driven to an undesired region of the ventilator circuit 1110. Furthermore, the combination of a droplet forming nebulizer and an aerosol introducer 1145 as described is beneficial in that there is a reduction in the variability of dosing when the ventilator uses different tidal volumes, thus making the system more universal.

The volume of the first channel 1265, that is, the volume of the portion of the adapter 1145 that receives the aerosolized pharmaceutical formulation and through which inhalation air flows, may be selected so that the aerosol delivery efficiency is increased for a particular ventilator and/or aerosolizer. For example, in one or more versions of Figs. 18A-18C, the volume of the first channel 1265, which includes the volume extending from the one-way valve 1270 to a junction with the second channel 1260, may be from about 10 ml to about 1000 ml, such as from about 125 ml to about 500 ml or from about 200 ml to about 300 ml. In this regard, the volume of the first channel 1265 extending from the one-way valve 1270 downstream to the end of the adapter 1145, generally ranges from about 5 ml to about 500 ml, such as from about 50 ml to about 150 ml, or about 60 ml to about 100 ml. When the adapter 1145 is being used in conjunction with a jet nebulizer, it may be desirable to have a larger first channel volume. Jet nebulizers introduce compressed air into the ventilator circuit, and a larger first channel volume would reduce the impact of this introduction. Accordingly, it has been found that for jet nebulizer use, the first channel volume may be from about 50 ml to about 1000 ml, such as about 100 ml to about 500 ml, about 150 ml to about 250 ml, or about 200 ml. For vibrating mesh nebulizers, such as the Aeroneb® Pro and the PARI eFlow, reproducible administrations can result from smaller first channel volumes. It has been determined, for example, that the first channel volume for an adapter 1145 used with a vibrating mesh nebulizer may be any volume greater than about 10 ml, such as from about 10 ml to about 1000 ml, about 50 ml to about 200 ml, or about 90 ml. Both the stored volume and valving affect the performance of the present invention.

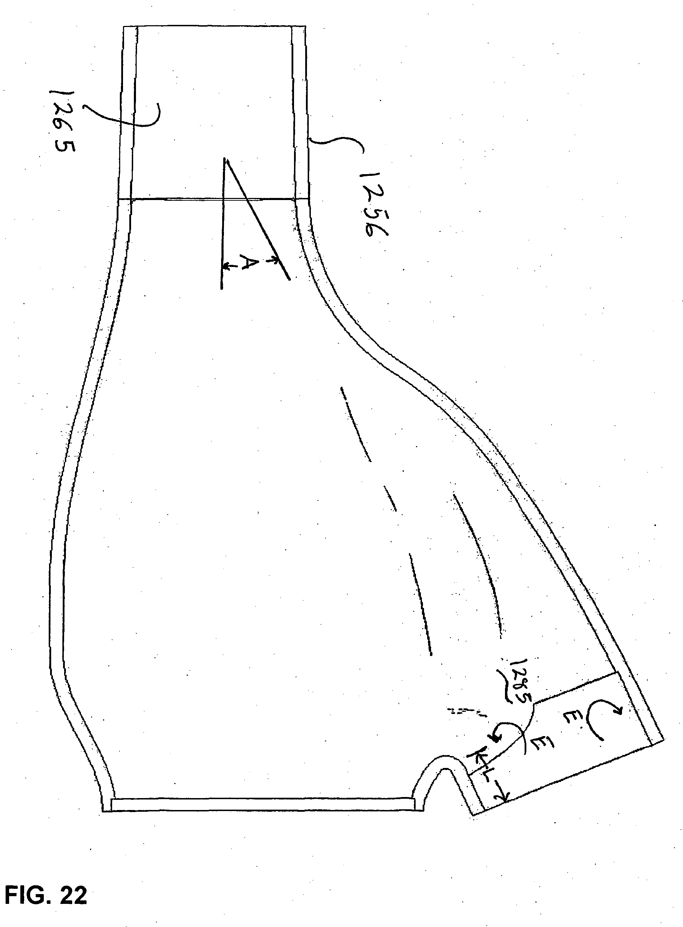

The first channel 1265 and extension portion 1285 may assume various shapes. For example, the extension portion 1285 may form an angle, A (see Fig. 22), such as about 10 degrees to about 70 degrees, about 20 degrees to about 60 degrees, or about 30 degrees to about 40 degrees, relative to an axis of the first channel 1265. Such an angle may affect the likelihood that the droplets are entrained in the inhalation gases.

The extension portion 1285 may also be angled relative to an axis of the first channel 1265 out of the plane of Fig. 22. As a result of this angle, the droplets may follow a helical path through the first channel. Such a helical path may affect the likelihood that the droplets are entrained in the inhalation gases.

The extension portion 1285 may also include one or more one-way check valves (not shown) to atmosphere. The one-way check valves would allow air into the extension portion when the nebulizer is operating to minimize the formation of eddies.

The length of the extension portion 1285 may also vary. For instance, a length, L, of the shortest portion of the extension portion 1285 may range from about 0 mm to about 5 cm, such as about 5 mm to about 2.5 cm, or about 1 cm to about 2 cm. As discussed below, the length of the extension portion may have an effect on the likelihood that the droplets are entrained in the inhalation gases.

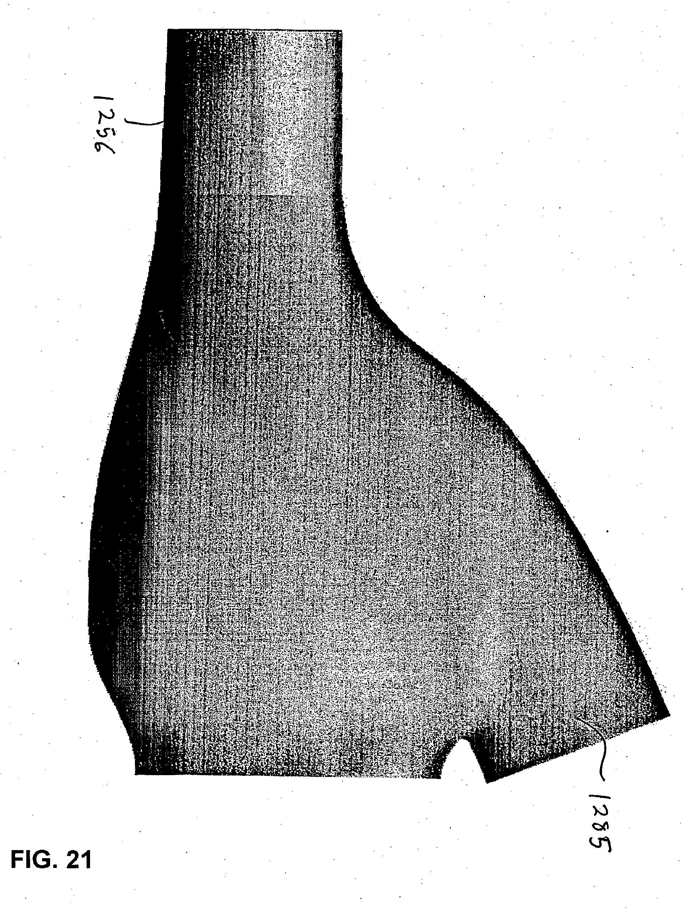

Figs. 21 and 22 show an embodiment of the first tube 1256 and channel 1265 that may, e.g., be used with a vibrating mesh nebulizer. The first channel 1265 includes an extension portion 1285 that is in communication with the aerosolization apparatus 1150 and is able to receive aerosolized pharmaceutical formulation. In this case, the length, L, of a short portion the extension portion is about 2 cm. In some embodiments, when used with a vibrating mesh nebulizer, many droplets may condense on the wall of the extension portion 1285. It was discovered that droplets may entrain air and create eddies, E. The eddies then cause many droplets to strike the wall of the extension portion 1285 and first channel 1265.

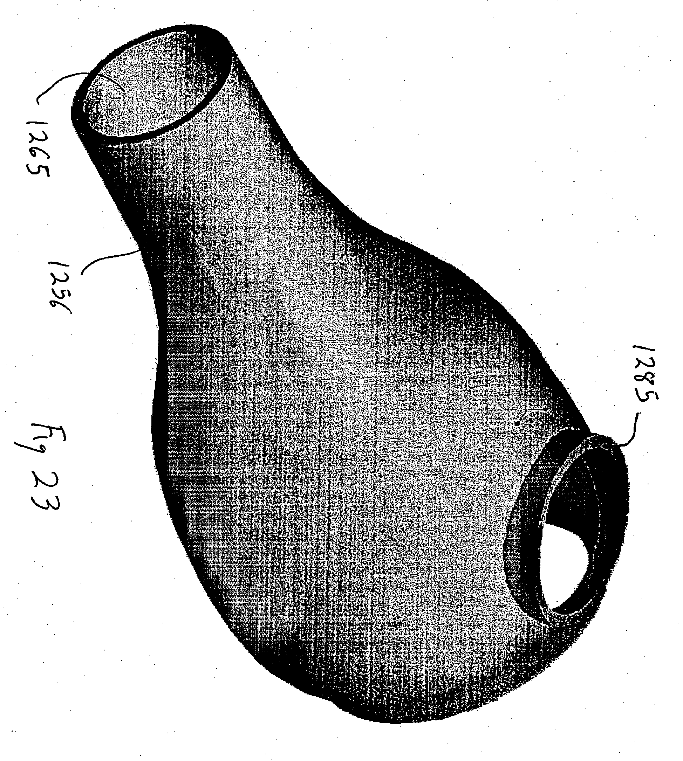

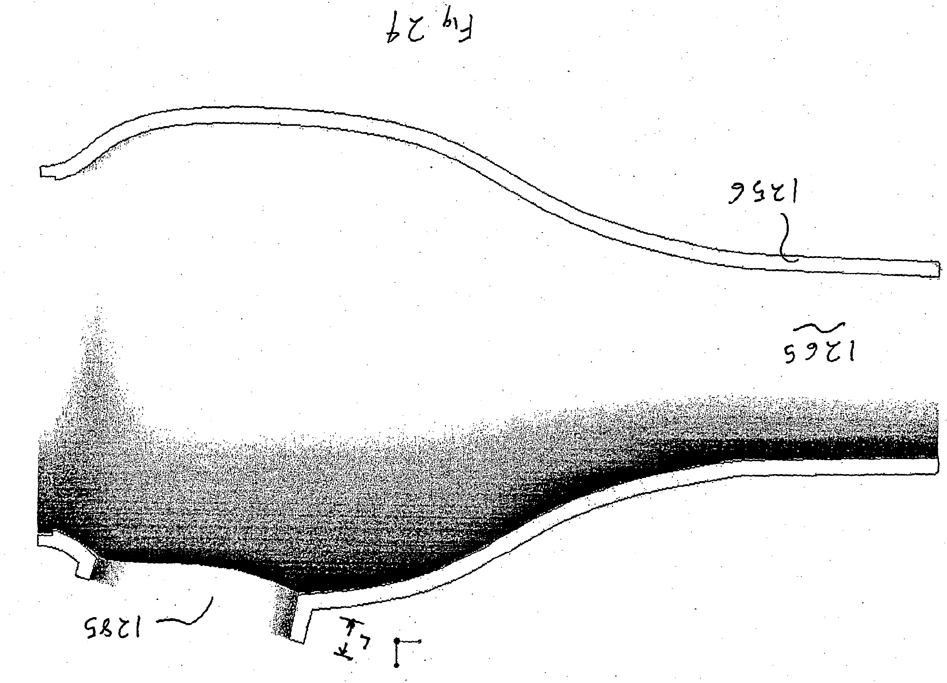

Figs. 23 and 24 show another embodiment of the first channel 1265 that may, e.g., be used with a vibrating mesh nebulizer. The first channel 1265 includes an extension portion 1285 that is in communication with the aerosolization apparatus 1150 and is able to receive aerosolized pharmaceutical formulation. In this case, the length, L, of a short portion the extension portion is about 0.001 cm to about 1 cm, such as about 0.1 cm to about 0.5 cm. When this embodiment was used with a vibrating mesh nebulizer, advantageously fewer droplets condensed on the wall of the extension portion 1285 and first channel 1265 than in the case of embodiments with longer extension portions 1285.

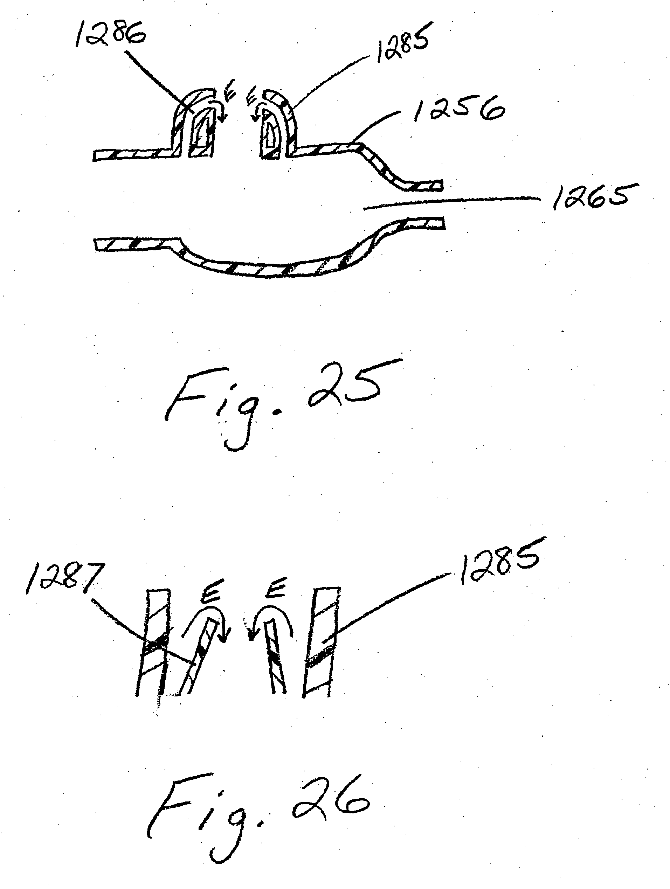

Fig. 25 is a side sectional view showing an embodiment wherein the extension portion 1285 comprises one or more channels 1286. It is expected that, when used with a nebulizer, the one or more channels 1286 would facilitate the formation of eddies, E, that minimize the likelihood of droplet condensation.

Fig. 26 shows an embodiment wherein the extension portion 1285 contains a cone-shape sheath 1287. It is expected that, when used with a nebulizer, the sheath 1287 would facilitate the formation of eddies, E, that minimize the likelihood of droplet condensation. The sheath 1287 typically comprises a continuous surface or may comprise a discontinuous surface.

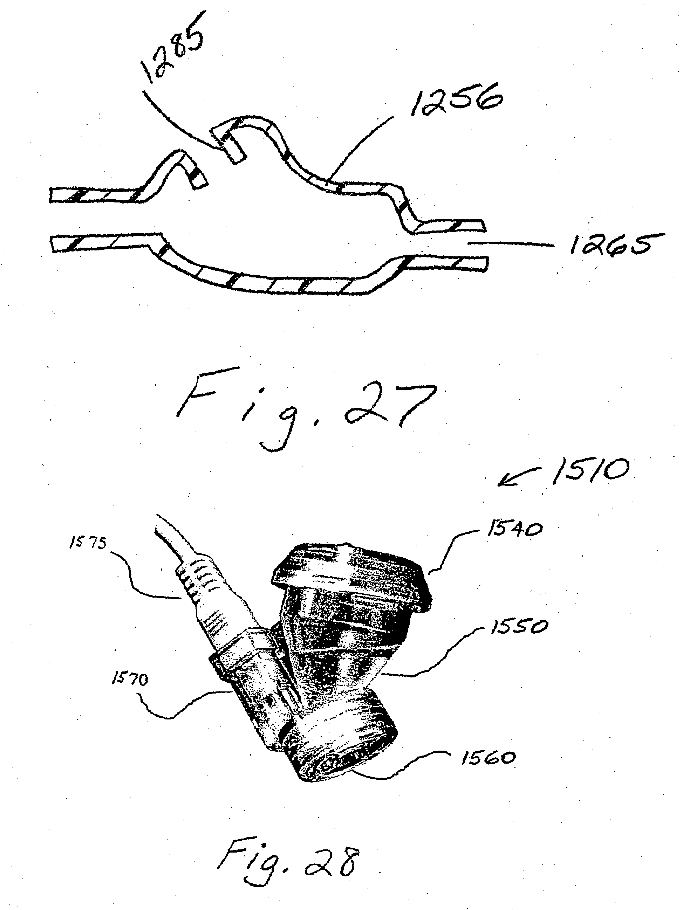

Fig. 27 shows an embodiment in which the extension portion 1285 is inverted, i.e, extends into the interior of the channel 1265. It is expected that, when used with a nebulizer, the inverted extension portion 1285 would facilitate the formation of eddies, E, that minimize the likelihood of droplet condensation.

Described are nebulizers and nebulizer systems. In one or more arrangements, the drug dose (or multi-dose) container includes one or more aerosol generating elements. For instance, a vial or other containment device, may be combined with the aperture plate or mesh used as the primary aerosol generating element in a vibrating mesh nebulizer. It should be noted, however, that the nebulizers and nebulizer systems are not limited to use in ventilator circuits, and in fact may be used in any application where jet, ultrasonic, vibrating mesh, or other nebulizer might be used, such as with a continuous positive airway pressure (CPAP) device, or a non-invasive ventilation or breathing assistance system.

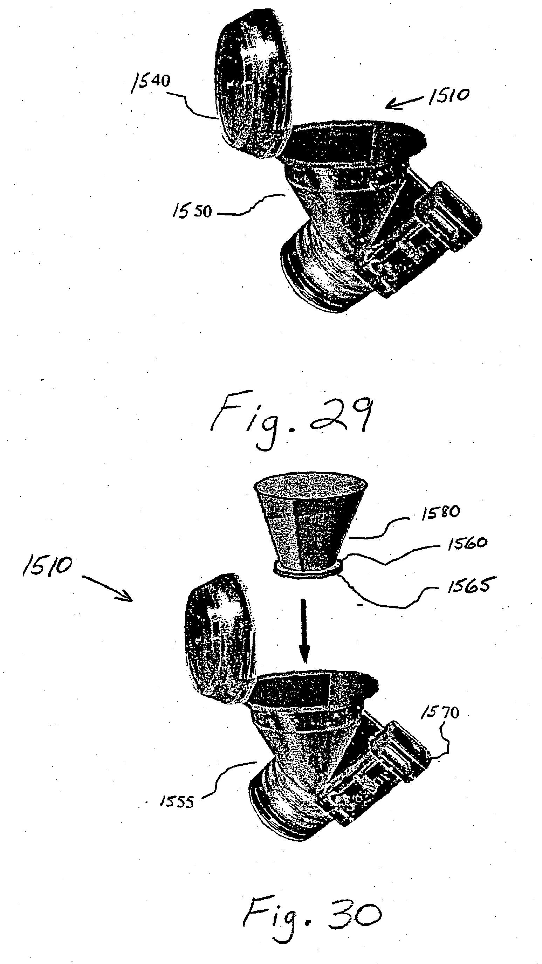

Figs. 28 and 29 show a vibrating mesh nebulizer 1510 used in ventilator circuits for mechanically ventilated patients. To administer liquid drug formulation, cap 1540 is opened. Liquid drug formulation is poured into drug holding chamber 1550 where the drug formulation comes into contact with vibrating mesh element 1560. An appropriate signal, such as an electronic signal, which may be a sinusoidal, square or other waveform of specified amplitude and frequency, is delivered via cable 1575 connected to cable receptacle 1570 to provide electronic signal to vibrating mesh element 1560 in order to deliver liquid drug formulation in the form of aerosol into the ventilator circuit to the patient. Fig. 29 shows the embodiment with cap 1540 open to receive liquid drug formulation into drug holding chamber 1550.

Fig. 30 shows another vibrating mesh nebulizer 1510. The liquid drug container or vial 1580 is incorporated with vibrating mesh element 1560 with integral piezoelectric element 1565 and is designed to fit into vial receiver 1555. The vibrating mesh element 1560 includes the piezoelectric element that serves to introduce vibration of proper frequency into vibrating mesh element 1560. An appropriate signal, such as an electronic signal is delivered via cable 1575 connected to cable receptacle 1570 to provide electronic signal to vibrating mesh element 1560. Note that signals other than electrical may be used in any embodiment that benefits from such a signal. Thus optical, RP, thermal, magnetic, mechanical and others may be used. Accordingly, in some arrangements, the cable 1575 is unneeded.

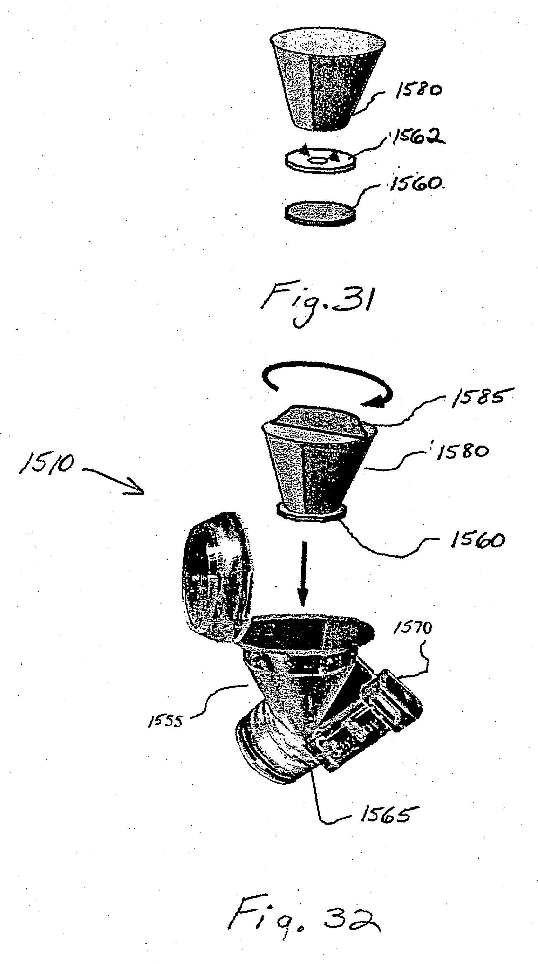

Fig. 31 shows vial rupture element 1562. The rupture element 1562 is activated on insertion of the vial, bringing drag formulation into contact with the aperture plate. The rupture element may be on the vial or on the body.

In another arrangement, shown in Fig. 32, the piezoelectric element 1565 that serves to introduce vibration of proper frequency into vibrating mesh element 1560 is contained within the structure of the vibrating mesh nebulizer in or near to vial receiver 1555 and transmits the vibration of proper frequency into vibrating mesh element 1560 through mechanical contact. Proper electronic signal, which may be a sinusoidal, square or other waveform of a specified amplitude and frequency, is delivered via cable 1575 connected to cable receptacle 1570 to provide electronic signal to piezoelectric element 1565 that serves to introduce vibration of proper frequency into vibrating mesh element 1560. Twist handle 1585 may be incorporated in order to facilitate the application of torque for ensuring electrical and/or mechanical contact.

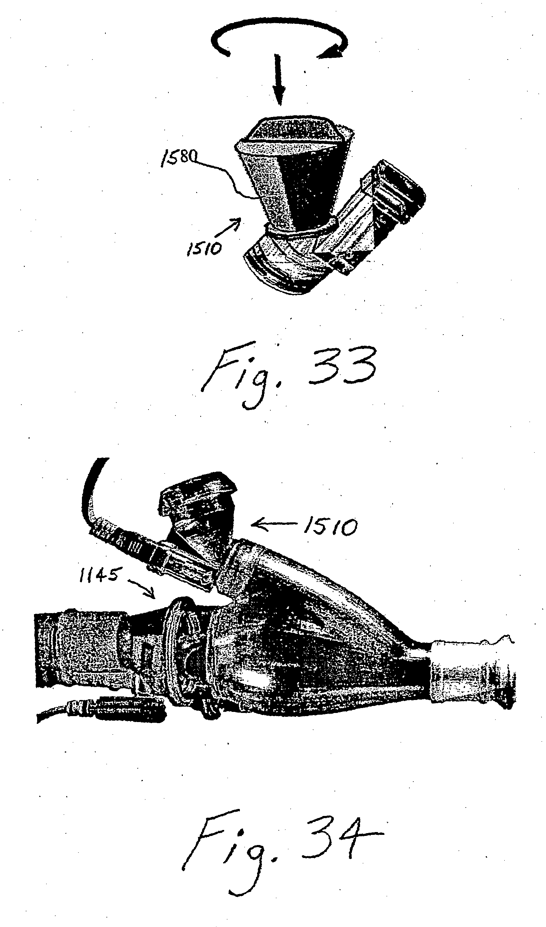

In Fig. 33, the vial receiver 1555 of previous arrangements is omitted, and vial 1580 serves as liquid drug container and as a support. Alternatively, the vial receiver 1555 serves as the support.

Fig. 34 shows a vibrating mesh nebulizer system 1510 employed with an adapter 1145.

In view of the above, in one or more embodiments, the nebulizer systems provide consistent, safe and convenient pulmonary delivery of medication. In one or more embodiments, the nebulizer systems provide (1) simple insertion of medication cartridge, instead of measuring and pouring of medication into a cavity for administration; (2) a vibrating mesh element integral with the medication cartridge, thereby eliminating the need for mesh cleaning for subsequent use; (3) simplified mesh manufacturing, since limited or single use mesh need not be protected from corrosion induced by extended drug contact and related chemical interaction; and/or (4) protection against potentially dangerous off-label use through the integration of drug vial and mesh element into a unified, inseparable single-use drug-mesh unit.

Fig. 35 is a partial schematic view of a ventilator circuit which includes the adapter of the present invention. Arrows E show air flowing from a ventilator. The air passes through an inhalation line 1115 toward the patient. The inhalation line 1115 may include a heated wire humidifier 1300, which can be controlled by using a temperature sensor located in temperature probe port 1240. The air then passes through one-way inhalation valve 1270. Pharmaceutical formulation may be added through extension portion 1285. The air then passes through inhalation vent tubing 1310 before passing into a Y-piece 1320. The Y-piece 1320 often has a length of up to about 1 m, such as up to about 0.5 m. The Y-piece 1320 may be connected to an endotracheal tube (not shown), which is inserted into a patient.

The exhalation air passes through the endotracheal tube into the Y-piece 1320. As a result of the valving, the exhalation air then passes through exhalation vent tubing 1330. The air then passes through one-way exhalation valve 1290 and then out through exhalation line 1120 toward a ventilator, as shown by arrows F.

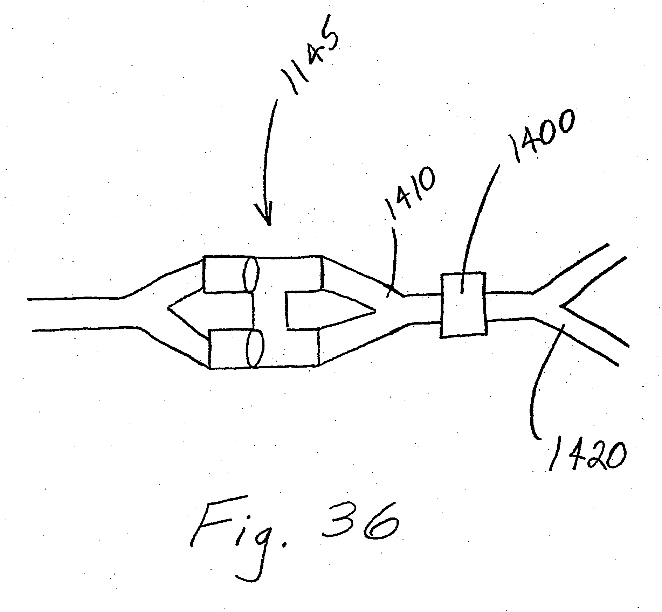

Fig. 36 schematically shows an embodiment that is similar to the one shown in Fig. 35, except that it also includes a heat/moisture exchange (HME) filter 1400. The HME filter 1400 removes moisture from exhalation gases and adds moisture to inhalation gases. To accommodate the HME filter, the ventilator circuit includes two Y-tubes 1410 and 1420.

The valves and devices of the present invention may be made by any of the various methods and techniques known and available to those skilled in the art.

### EXPERIMENTAL

Tables 1 and 2 below are 5 day use life studies, showing the reliability of valves and adapters of the present invention after running for 5 days, administering an antibiotic under simulated conditions. As can be seen, the percentage delivered at the inspiratory line did not change significantly over time, thus evidencing non-sticking of the valves and/or good flow properties within the adapter. In the tests, a jet nebulizer, a cloverleaf valve, and an adapter in accordance with one or more embodiments of the present invention were employed. Table 1 shows results for gentamicin, while Table 2 shows results for vancomycin.

**Table 1**

| | | | | | | | | | | |
|---|---|---|---|---|---|---|---|---|---|---|
| **Formulation** | | | Gentamicin: Device 8022A - in WFI, Device 8022B - in WFI | | | | | | | |
| **Nebulizer** | | | AeroTechll | | | | | | | |
| **Solution Strength** | | | 120.0 mg/ml | | | | | | | |
| **TV/RR/PFIR/BIAS/HUM/NEB:** | | | 600/12/60/6/ON/CN | | | | | | | |
| **Fill Volume** | | | 5 ml | | | | | | | |

| **Device ID** | **Run** | **Sample label** | **CONTENT [mg]** | **% CONTENT (%)** | **Comments** | **MEAN** | **MEAN CONTENT [mg]** | **SD CONTENT [mg]** | **MEAN % CONTENT (%)** | **SD % CONTENT (%)** |
|---|---|---|---|---|---|---|---|---|---|---|
| 8022A | Day 1 | Inspiratory | 94.6 | 15.8% | . | Inspiratory | 95.0 | 5.7 | 15.8% | 0.9% |
| | | Nebulizer | 201.6 | 33.6% | . | Nebulizer | 190.5 | 13.2 | 31.7% | 2.2% |
| | | Recovery | 296.2 | 49.4% | . | Recovery | 285.5 | 17.3 | 47.6% | 2.9% |
| | Day 3 | Inspiratory | 89.6 | 14.9% | . | | | | | |
| | | Nebulizer | 175.9 | 29.3% | . | | | | | |
| | | Recovery | 265.5 | 44.2% | . | | | | | |
| | Day 5 | Inspiratory | 100.9 | 16.8% | . | | | | | |
| | | Nebulizer | 193.9 | 32.3% | . | | | | | |
| | | Recovery | 294.8 | 49.1% | . | | | | | |
| | Final Adapter | Adapter | 0.2 | 0.0% | . | | | | | |
| 8022B | Day 1 | Inspiratory | 87.5 | 14.6% | . | Inspiratory | 99.0 | 10.5 | 16.5% | 1.8% |
| | | Nebulizer | 233.2 | 38.9% | . | Nebulizer | 228.2 | 44.7 | 38.0% | 7.5% |
| | | Recovery | 320.6 | 53.4% | . | Recovery | 327.2 | 41.6 | 54.5% | 6.9% |
| | Day 3 | Inspiratory | 101.5 | 16.9% | . | | | | | |
| | | Nebulizer | 270.3 | 45.0% | . | | | | | |
| | | Recovery | 371.7 | 62.0% | . | | | | | |
| | Day 5 | Inspiratory | 108.1 | 18.0% | . | | | | | |
| | | Nebulizer | 18.3 | 30.2% | . | | | | | |
| | | Recovery | 289.4 | 48.2% | . | | | | | |
| | Final Adapter | Adapter | 0.3 | 0.1% | . | | | | | |

**Table 2**

| | | | | | | | | | | |
|---|---|---|---|---|---|---|---|---|---|---|
| **Formulation** | | | Vancomycin: Device 8022A - in WFI, Device 8022B - in Quarter Normal Saline | | | | | | | |
| **Nebulizer** | | | AeroTechII | | | | | | | |
| **Solution Strength** | | | 120.0 mg/ml | | | | | | | |
| **TV/RR/PFIR/BIAS/HUM/NEB:** | | | 600/12/6016/ON/CN | | | | | | | |
| **Fill Volume** | | | 5 ml | | | | | | | |

| **Device ID** | **Run** | **Sample label** | **CONTENT [mg]** | **% CONTENT (%)** | **comments** | **MEAN** | **MEAN CONTENT [mg]** | **SD CONTENT [mg]** | **MEAN % CONTENT (%)** | **SD% CONTENT (%)** |
|---|---|---|---|---|---|---|---|---|---|---|
| 8022A | Day 1 | Inspiratory | 93.9 | 15.7% | . | Inspiratory | 96.6 | 4.6 | 16.1% | 0.8% |
| | | Nebulizer | 221.9 | 37.0% | . | Nebulizer | 223.3 | 1.6 | 37.2% | 0.3% |
| | | Recovery | 315.8 | 52.6% | . | Recovery | 319.9 | 6.1 | 53.3% | 1.0% |
| | Day 3 | Inspiratory | 93.9 | 15.6% | . | | | | | |
| | | Nebulizer | 223.1 | 37.2% | . | | | | | |
| | | Recovery | 316.9 | 52.8% | . | | | | | |
| | Day 5 | Inspiratory | 101.9 | 17.0% | . | | | | | |
| | | Nebulizer | 225.0 | 37.5% | . | | | | | |
| | | Recovery | 327.0 | 54.5% | . | | | | | |
| | Final Adapter | Adapter | 2.7 | 0.5% | . | | | | | |
| 8022B | Day 1 | Inspiratory | 72.0 | 12.0% | . | Inspiratory | 72.5 | 6.5 | 12.1% | 1.1% |
| | | Nebulizer | 275.9 | 46.0% | . | Nebulizer | 266.9 | 28.9 | 44.5% | 4.8% |
| | | Recovery | 347.9 | 58.0% | . | Recovery | 339.5 | 22.5 | 56.6% | 3.8% |
| | Day 3 | Inspiratory | 79.3 | 13.2% | . | | | | | |
| | | Nebulizer | 234.7 | 39.1% | . | | | | | |
| | | Recovery | 314.0 | 52.3% | . | | | | | |
| | Day 5 | Inspiratory | 66.3 | 11.0% | . | | | | | |
| | | Nebulizer | 290.3 | 48.4% | . | | | | | |
| | | Recovery | 356.6 | 59.4% | . | | | | | |
| | Final Adapter | Adapter | 0.3 | 0.1% | . | | | | | |

Additional results showed that adapters and valve configurations of the present invention can deliver a higher inspiratory dose, and/or less dose variation (smaller max/min ratio) and/or affected by fewer factors including minute respiration, respiratory rate, inspiratory flow rate, bias flow and humidity, all compared to that of prior art configurations.

The pharmaceutical formulation may comprise an active agent (or medicament) for administration to the respiratory tract of the user. The active agent described herein includes an agent, drug, compound, composition of matter or mixture thereof which provides some pharmacologic, often beneficial, effect. This includes foods, food supplements, nutrients, drugs, vaccines, vitamins, and other beneficial agents. As used herein, the terms further include any physiologically or pharmacologically active substance that produces a localized or systemic effect in a patient. An active agent for incorporation in the pharmaceutical formulation described herein may be an inorganic or an organic compound, including, without limitation, drugs which act on: the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system, and the central nervous system.

In one particular example, the pharmaceutical formulation comprises an antibiotic for administration to a ventilated patient to treat or prevent ventilator associated pneumonia. Such administration is described in aforementioned Smaldone et al PCT Patent Application entitled "Methods, Devices and Formulations for Targeted Endobronchial Therapy" WO 2004/071368, filed May 7, 2003; in Smaldone et al U.S. Patent Application 10/430,765, filed on May 6, 2003; in Smaldone et al, U.S. Patent Application 10/430,658, filed on May 6, 2003; and in U.S. Provisional Patent Applications 60/378,475; 60/380,783; 60/420,429; 60/439,894; and 60/442,785. Using an adapter 1145 according to the present invention in connection with the administration of aerosolized antibiotics offers substantial benefits. For example, when using the adapter 1145 of the invention, substantially less pharmaceutical formulation is lost to the environment which results in a reduction in bacterial resistance against the antibiotic. In addition, the adapter 1145 is able to deliver a more consistent dose which is particularly useful for antibiotic therapy. In one particular version, the pharmaceutical formulation may comprise vancomycin and/or gentamicin. Additional examples of the pharmaceutical formulation are disclosed in commonly-owned U.S. Provisional Application Serial No. 60/722,564, filed September 29, 2005 (Attorney Docket No. 0280.PRO), "Antibiotic Formulations, Unit Doses, Kits, and Methods".

Alternatively or additionally, suitable active agents may be selected from, for example, hypnotics and sedatives, psychic energizers, tranquilizers, respiratory drugs, anticonvulsants, muscle relaxants, antiparkinson agents (dopamine antagnonists), analgesics, antiinflammatories, antianxiety drugs (anxiolytics), appetite suppressants, antimigraine agents, muscle contractants, anti-mfectives (antibiotics, antivirals, antifungals, vaccines) antiarthritics, antimalarials, antiemetics, anepileptics, bronchodilators, cytokines, growth factors, anti-cancer agents, antithrombotic agents, antihypertensives, cardiovascular drugs, antiarrhythmics, antioxicants, anti-asthma agents, hormonal agents including contraceptives, sympathomimetics, diuretics, lipid regulating agents, antiandrogenic agents, antiparasitics, anticoagulants, neoplasties, antineoplastics, hypoglycemics, nutritional agents and supplements, growth supplements, antienteritis agents, vaccines, antibodies, diagnostic agents, and contrasting agents. The active agent, when administered by inhalation, may act locally or systemically, or in combination.

The active agent may fall into one of a number of structural classes, including but not limited to small molecules, peptides, polypeptides, proteins, polysaccharides, steroids, proteins capable of eliciting physiological effects, nucleotides, oligonucleotides, polynucleotides, fats, electrolytes, and the like.

Examples of active agents suitable for use in this invention include but are not limited to one or more of bronchodilators, such as β-2 agonists (such as albuterol/salbutamol, theophylline, formoterol, salmeterol, indecaterol), anti-muscarinics and anti-cholinergics, Tiotropium, mast cell stablizers, steroids (e.g., fluticasone, mometasone, ciclesonide), drugs to slow the recruitment of inflammatory cells, PDE4 inhibitors, immunosuppressive drugs (like cyclosporin, tacrolimus, pimecrolimus, etc), anti-fibrotic agents, elastase inhibiting agents (alpha-1 antitrypsin), agents designed to adjust tonicity, agents that stimulate natural processes to drive reduction and removal excess fluid from the lung, surfactants of all forms, calcitonin, amphotericin B, echinocandins (e.g., Cancidas from Merck, or Anidulafungin from Pfizer), erythropoietin (EPO), Factor VIII, Factor IX, ceredase, cerezyme, cyclosporin, granulocyte colony stimulating factor (GCSF), thrombopoietin (TPO), alpha-1 proteinase inhibitor, elcatonin, granulocyte macrophage colony stimulating factor (GMCSF), growth hormone, human growth hormone (HGH), growth hormone releasing hormone (GHRH), heparin, low molecular weight heparin (LMWH), interferon alpha, interferon beta, interferon gamma, interleukin-1 receptor, interleukin-2, interleukin-1 receptor antagonist, interleukin-3, interleukin-4, interleukin-6, luteinizing hormone releasing hormone (LHRH), factor IX, insulin, pro-insulin, insulin analogues (e.g., mono-acylated insulin as described in U.S. Patent No. 5,922,675), amylin, C-peptide, somatostatin, somatostatin analogs including octreotide, vasopressin, follicle stimulating hormone (FSH), insulin-like growth factor (IGF), insulintropin, macrophage colony stimulating factor (M-CSF), nerve growth factor (NGF), tissue growth factors, keratinocyte growth factor (KGF), glial growth factor (GGF), tumor necrosis factor (TNF), endothelial growth factors, parathyroid hormone (PTH), glucagon-like peptide thymosin alpha 1, IIb/IIIa inhibitor, phosphodiesterase (PDE) compounds, VLA-4 inhibitors, bisphosponates, respiratory syncytial virus antibody, cystic fibrosis transmembrane regulator (CFTR) gene, deoxyreibonuclease (Dnase), bactericidal/permeability increasing protein (BPI), anti-CMV antibody, 13-cis retinoic acid, macrolides such as erythromycin, oleandomycin, troleandomycin, roxithromycin, clarithromycin, davercin, azithromycin, flurithromycin, dirithromycin, josamycin, spiromycin, midecamycin, leucomycin, miocamycin, rokitamycin, andazithromycin, and swinolide A; fluoroquinolones such as ciprofloxacin, ofloxacin, levofloxacin, trovafloxacin, alatrofloxacin, moxifloxicin, norfloxacin, enoxacin, grepafloxacin, gatifloxacin, lomefloxacin, sparfloxacin, temafloxacin, pefloxacin, amifloxacin, fleroxacin, tosufloxacin, prulifloxacin, irloxacin, pazufloxacin, clinafloxacin, and sitafloxacin, aminoglycosides such as gentamicin, netilmicin, paramecin, tobramycin, amikacin, kanamycin, neomycin, and streptomycin, vancomycin, teicoplanin, rampolanin, mideplanin, colistin, daptomycin, gramicidin, colistimethate, polymixins such as polymixin B, capreomycin, bacitracin, penems; penicillins including penicllinase-sensitive agents like penicillin G, penicillin V, penicillinase-resistant agents like methicillin, oxacillin, cloxacillin, dicloxacillin, floxacillin, nafcillin; gram negative microorganism active agents like ampicillin, amoxicillin, and hetacillin, cillin, and galampicillin; antipseudomonal penicillins like carbenicillin, ticarcillin, azlocillin, mezlocillin, and piperacillin; cephalosporins like cefpodoxime, cefprozil, ceftbuten, ceftizoxime, ceftriaxone, cephalothin, cephapirin, cephalexin, cepliradrine, cefoxitin, cefamandole, cefazolin, cephaloridine, cefaclor, cefadroxil, cephaloglycin, cefuroxime, ceforanide, cefotaxime, cefatrizine, cephacetrile, cefepime, cefixime, cefonicid, cefoperazone, cefotetan, cefmetazole, ceftazidime, loracarbef, and moxalactam, monobactams like aztreonam; and carbapenems such as imipenem, meropenem, pentamidine isethiouate, albuterol sulfate, lidocaine, metaproterenol sulfate, beclomethasone diprepionate, triamcinolone acetamide, budesonide acetonide, fluticasone, ipratropium bromide, flunisolide, cromolyn sodium, ergotamine tartrate and where applicable, analogues, agonists, antagonists, inhibitors, and pharmaceutically acceptable salt forms of the above. In reference to peptides and proteins, the invention is intended to encompass synthetic, native, glycosylated, unglycosylated, pegylated forms, and biologically active fragments and analogs thereof.

Active agents for use in the invention further include nucleic acids, as bare nucleic acid molecules, vectors, associated viral particles, plasmid DNA or RNA or other nucleic acid constructions of a type suitable for transfection or transformation of cells, i.e., suitable for gene therapy including antisense. Further, an active agent may comprise live attenuated or killed viruses suitable for use as vaccines. Other useful drugs include those listed within the Physician's Desk Reference (most recent edition).

The active agents also include any and all combinations of all of the above, and metabolites, different chiral forms, salts, free base forms, or enantiomers of the above.

The amount of active agent in the pharmaceutical formulation will be that amount necessary to achieve a desired result, such as to deliver a prophylactically or therapeutically effective amount of the active agent per unit dose to achieve the desired result. In practice, this will vary widely depending upon the particular agent, its activity, the severity of the condition to be treated, the patient population, dosing requirements, and the desired therapeutic effect. The composition will generally contain anywhere from about 1% by weight to about 99% by weight active agent, typically from about 2% to about 95% by weight active agent, and more typically from about 5% to 85% by weight active agent, and will also depend upon the relative amounts of additives contained in the composition. The are particularly useful for active agents that are delivered in doses of from 0.001 mg/day to 100 mg/day, such as in doses from 0.01 mg/day to 75 mg/day, or in doses from 0.10 mg/day to 50 mg/day. It is to be understood that more than one active agent may be incorporated into the formulations described herein and that the use of the term "agent" in no way excludes the use of two or more such agents.

The pharmaceutical formulation may comprise a pharmaceutically acceptable excipient or carrier which may be taken into the lungs with no significant adverse toxicological effects to the subject, and particularly to the lungs of the subject. In addition to the active agent, a pharmaceutical formulation may optionally include one or more pharmaceutical excipients which are suitable for pulmonary administration. These excipients, if present, are generally present in the composition in amounts ranging from about 0.01 wt% to about 95 wt%, such as about 0.5 wt% to about 80 wt%, or about 1 wt% to about 60 wt%. Generally, such excipients will, in part, serve to further improve the features of the active agent composition, for example by providing more efficient and reproducible delivery of the active agent, improving the handling characteristics of powders, such as flowability and consistency, and/or facilitating manufacturing and filling of unit dosage forms. In particular, excipient materials can often function to further improve the physical and chemical stability of the active agent, minimize the residual moisture content and hinder moisture uptake, and to enhance particle size, degree of aggregation, particle surface properties, such as rugosity, ease of inhalation, and the targeting of particles to the lung. One or more excipients may also be provided to serve as bulking agents when it is desired to reduce the concentration of active agent in the formulation.

Pharmaceutical excipients and additives useful in the present pharmaceutical formulation include but are not limited to amino acids, peptides, proteins, non-biological polymers, biological polymers, carbohydrates, such as sugars, derivatized sugars such as alditols, aldonic acids, esterified sugars, and sugar polymers, which may be present singly or in combination. Suitable excipients are those provided in WO 96/32096. The excipient may have a glass transition temperature (Tg) above about 35°C, such as above about 40°C, above about 45°C, or above about 55°C.

Exemplary protein excipients include albumins such as human serum albumin (HSA), recombinant human albumin (rHA), gelatin, casein, hemoglobin, and the like. Suitable amino acids (outside of the dileucyl-peptides of the invention), which may also function in a buffering capacity, include alanine, glycine, arginine, betaine, histidine, glutamic acid, aspartic acid, cysteine, lysine, leucine, isoleucine, valine, methionine, phenylalanine, aspartame, tyrosine, tryptophan, and the like. Preferred are amino acids and polypeptides that function as dispersing agents. Amino acids falling into this category include hydrophobic amino acids such as leucine, valine, isoleucine, tryptophan, alanine, methionine, phenylalanine, tyrosine, histidine, and proline. Dispersibility- enhancing peptide excipients include dimers, trimers, tetramers, and pentamers comprising one or more hydrophobic amino acid components such as those described above.

Carbohydrate excipients suitable for use in the invention include, for example, monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol), pyranosyl sorbitol, myoinositol and the like.

The pharmaceutical formulation may also include a buffer or a pH adjusting agent, typically a salt prepared from an organic acid or base. Representative buffers include organic acid salts of citric acid, ascorbic acid, gluconic acid, carbonic acid, tartaric acid, succinic acid, acetic acid, or phthalic acid, Tris, tromethamine hydrochloride, or phosphate buffers.

The pharmaceutical formulation may also include polymeric excipients/additives, e.g., polyvinylpyrrolidones, derivatized celluloses such as hydroxymethylcellulose, hydroxyethylcellulose, and hydroxypropylmethylcellulose, Ficolls (a polymeric sugar), hydroxyethylstarch, dextrates (e.g., cyclodextrins, such as 2-hydroxypropyl-β-cyclodextrin and sulfobutylether-β-cyclodextrin), polyethylene glycols, and pectin.

The pharmaceutical formulation may further include flavoring agents, taste-masking agents, inorganic salts (for example sodium chloride), antimicrobial agents (for example sodium chloride), antimicrobial agents (for example benzalkonium chloride), sweeteners, antioxidants, antistatic agents, surfactants (for example polysorbates such as "TWEEN 20" and "TWEEN 80"), sorbitan esters, lipids (for example phospholipids such as lecithin and other phosphatidylcholines, phosphatidylethanolamines), fatty acids and fatty esters, steroids (for example cholesterol), and chelating agents (for example EDTA, zinc and other such suitable cations). Other pharmaceutical excipients and/or additives suitable for use in the compositions according to the invention are listed in "Remington: The Science & Practice of Pharmacy", 19th ed., Williams & Williams, (1995), and in the "Physician's Desk Reference", 52nd ed., Medical Economics, Montvale, NJ (1998).

In some cases, such as for MDI applications, the pharmaceutical formulation may also be treated so that it has high stability. Several attempts have dealt with improving suspension stability by increasing the solubility of surface-active agents in the HFA propellants. To this end U.S. Pat. No. 5,118,494, WO 91/11173 and WO 92/00107 disclose the use of HFA soluble fluorinated surfactants to improve suspension stability. Mixtures of HFA propellants with other perfluorinated cosolvents have also been disclosed as in WO 91/04011. Other attempts at stabilization involved the inclusion of nonfluorinated surfactants. In this respect, U.S. Pat. No. 5,492,688 discloses that some hydrophilic surfactants (with a hydrophilic/lipophilic balance greater than or equal to 9.6) have sufficient solubility in HFAs to stabilize medicament suspensions. Increases in the solubility of conventional nonfluorinated MDI surfactants (e.g. oleic acid, lecithin) can also reportedly be achieved with the use of co-solvents such as alcohols, as set forth in U.S. Pat. Nos. 5,683,677 and 5,605,674, as well as in WO 95/17195. Unfortunately, as with the prior art cosolvent systems previously discussed, merely increasing the repulsion between particles has not proved to be a very effective stabilizing mechanism in nonaqueous dispersions, such as MDI preparations. All of the aforementioned references being incorporated herein by reference in their entireties.

"Mass median diameter" or "MMD" is a measure of mean particle size, since the powders of the invention are generally polydisperse (i.e., consist of a range of particle sizes). MMD values as reported herein are determined by centrifugal sedimentation, although any number of commonly employed techniques can be used for measuring mean particle size. "Mass median aerodynamic diameter" or "MMAD" is a measure of the aerodynamic size of a dispersed particle. The aerodynamic diameter is used to describe an aerosolized powder in terms of its settling behavior, and is the diameter of a unit density sphere having the same settling velocity, generally in air, as the particle. The aerodynamic diameter encompasses particle shape, density and physical size of a particle. As used herein, MMAD refers to the midpoint or median of the aerodynamic particle size distribution of an aerosolized powder determined by cascade impaction.

In one or more versions, the powdered or liquid formulation for use in the present invention includes an aerosol having a particle or droplet size selected to permit penetration into the alveoli of the lungs, that is, typically less than about 10 µm mass median diameter (MMD), such as less than 7.5 µm, or less than 5 µm, and usually being in the range of 0.1 µm to 5 µm in diameter. When in a dry powder form, the pharmaceutical formulation may have a moisture content below about 10 wt%, such as below about 5 wt%, or below about 3 wt%. Such powders are described in WO 95/24183, WO 96/32149, WO 99/16419, and WO 99/16422.

The valves, adapters, systems, fittings, components and circuits of the present invention may be used in various systems, devices, apparatus, processes or methods wherein such valves, adapters, systems, fittings, components or circuits may result in a benefit. For example, the valves may be useful in any operation wherein a check valve is useful, especially where the valve provides at least on of minimum flow resistance, high reliability, low operating pressure and non-sticking operation. Thus, the uses of the valves, adapters, systems, fittings, components and circuits of the present invention are not particularly limited in their application.

As an example, the valves may be used with an adapter to facilitate delivery of a pharmaceutical formulation from an aerosolization device. The valves may be used with any of the pharmaceutical formulations, described above.

Active agents may be delivered simultaneously, some preferred order, and/or providing one agent in an aerosol of a certain size to target one region of the lung while providing another in another size to target another region. Thus, purposeful variation of the aerosol size we can cause some aerosol to deposit more proximally near the endotracheal tube to treat that area, while also sending in small aerosol to penetrate more deeply.

For instance, the present invention may be used in a method for treating or preventing pulmonary infections, including nosocomial infections, in animals, including, especially, humans. The method generally comprises administering to an animal subject or human patient in need thereof, as an aerosol, a prophylactically or therapeutically effective amount of an antibiotic substance or a pharmaceutically acceptable salt thereof. Several antibiotics may be delivered in combination or in seriatim. Preferably, the amounts delivered to the airways, if delivered systemically in such amounts, would not be sufficient to be therapeutically effective and would certainly not be enough to induce toxicity. At the same time, such amounts will result in sputum levels of antibiotic of more than about 10-100 times the minimum inhibitory concentration ("MIC").

In one or more aspects, the aerosolized particles are prevented from undergoing significant hygroscopic enlargement, since particles enrobed in water will tend to condense on the walls of an internal channel or surface. This method may comprise minimizing the opportunity for water contact with the aerosolized particles, or may comprise making the particles less hygroscopic, or both. In some arrangements, the method may involve reducing humidity in the ventilator circuit by a predetermined amount before nebulization begins. In some embodiments, the humidity may facilitate an MMAD of less than about 3 µm or less than about 1.5 µm. In other arrangements, each aerosol particle is delivered in contact with, such as encapsulated or enrobed in a substantially anhygroscopic material such as an envelope or capsule.

Of course, arrangements can be used where diameters are greater. Moreover, in some cases, there can be contemplated adjustments to the surface electrical charges on the particles or the walls. For example, assuming surface charge on the device is important, the connectors are made, or the Y piece is made, of metal (or at least coated with metal). Alternatively, the plastic connectors and/or Y piece can be treated with agents (e.g. wetting agents, detergents, soaps) to adjust surface charge.

In one or more aspects, the method comprises inserting an aerosol delivery end of the device within said patient's trachea to create a positioned device; and aerosolizing the pharmaceutical formulation under conditions such that said formulation is delivered through said aerosol delivery end of the device to the patient, wherein the aerosol first contacts the patient's trachea (thereby bypassing the oro-pharynx). The method may involve administering a mixture of antibiotics is particularly appropriate for intubated patients.

In one aspect, particular with respect to "constant-flow" ventilators, there can be contemplated limiting the delivery event strictly to the inspiratory phase of the ventilator cycle and, if possible, at a reduced flow-rate. Thus aerosolization may be actuated during (or in fixed relation to) the inspiration phase of the breathing cycle

It is not intended that the present invention on application should be limited to particular dosages. On the other hand, the efficiency of the aerosol systems and methods described herein permit amounts to be delivered that are too low to be generally effective if administered systemically, but are nonetheless effective amounts when administered in a suitable and pharmaceutically acceptable formulation directly to the airway. Importantly, while efficiencies can be increased, sometimes efficiencies are not increased at the expense of control over the dose. Thus, lower efficiencies are contemplated as preferred when delivery is more reproducible.

It is not intended that the present invention on application should be limited to use with antimicrobials that only kill or inhibit particular organisms. Various drugs and drug combinations that will address a wide variety of conditions caused by a wide variety of organisms. In one or more embodiments, there can be contemplated drugs or drug combinations effective in the treatment of infections caused by one or more of *P*. *aeruginosa, S*. *aureus, H. influenza,* and *S*. *pneumoniae, Acinetobacter species,* and/or antibiotic-resistant strains of bacteria such as methicillin-resistant *S*. *aureus,* among others are contemplated.

Of course, antivirals can also be aerosolized and administered in the manner of the antibiotic formulations of the present invention. This is particularly significant given the outbreak of severe acute respiratory syndrome (SARS).

While certain uses address infections, the improved aerosol systems and methods can be applied to any patient, human or animal, in need of an aerosol to the trachea and/or deep lung. For this reason, other drugs, or medicaments (e.g., steroids, proteins, peptides, nucleic acids, bronchodilator, surfactant, lidocaine, and the like) are contemplated as aerosols. Moreover other types of patients (e.g., cystic fibrosis, lung cancer, COPH, ARDS5 SAID, Heaves, respiratory infections, asthma, bronchospasm, and the like) are contemplated.

Moreover, while certain embodiments of the present invention are presented in the context of the intubated patient, other patients at risk for infection, whether intubated or not, are contemplated as treatable with the methods and devices of the present invention. For example, the elderly (particularly those in nursing homes), horses, dogs and cats in competitions (show and racing animals), animals that frequently travel (e.g., circus animals), animals in close quarters (e.g., zoos or farms), humans and animals in general are at risk for lung infections. The present invention relates to delivery of aerosols to the trachea and/or deep lung for such individuals-both prophylactically (i.e., before symptoms) and under acute conditions (i.e., after symptoms)--wherein said aerosols comprise antimicrobials, and in particular, the antibiotic mixtures described above.

In one or more aspects, there is contemplated administering the appropriate medication to a patient diagnosed with ARDS, IRDS, or chronic obstructive pulmonary disease (COPD).

The present invention is not limited to any precise desired outcome when using the above-described compositions, devices and methods. However, it is believed that use of the devices, of the present invention may result in a reduction in mortality rates of intubated patients, a decrease in the incidence of resistance (or at least no increase in resistance) because of the reduced systemic antibiotic exposure and elevated exposure at the targeted mucosal surface of the lung caused by local administration. As noted above, it is contemplated that the devices of the present invention are useful in the treatment of pneumonia (and may be more effective than systemic treatment--or at the very least, a useful adjunct). It is believed that related infections may also be prevented or reduced (e.g., prevention of sepsis, suppression of urinary tract infections, etc.)

Of course, a reduced use of systemic antibiotics because of the efficacy of the devices, of the present invention may result in reduced cost, reduced time on IV lines, and/or reduced time on central lines). Moreover, such a reduction should reduce antibiotic toxicity (as measured by reduced incidence of diarrhea and *C. difficile* infection, better nutrition, etc.)

It is believed that the devices of the present invention will locally result in a reduction of the ET/Trach tube biofilm. This should, in turn, get rid of secretions, decrease airway resistance, and/or decrease the work of breathing. The latter should ease the process of weaning the patient off of the ventilator.

The present invention contemplates specific embodiments that can replace commonly used elements of a ventilator system. A modular Y-piece attachable to a ventilator and to an endotracheal tube, wherein the modular Y-piece further comprises an aerosol generator, is contemplated. A lower arm [means what] of the modular Y piece may comprise the aerosol generator. While not limited to any precise desired outcome, it is contemplated that the modular Y-piece with integral generator will reduce the effects of the ventilator on all conventional aerosol systems (jet, ultrasonic and MDI), and at the same time enhance the positive qualities of a nebulization device such as an Aerogen™nebulizer. Again, while not limited to any precise desired outcome, it is contemplated that the modular Y-piece with integral generator will (1) reduce variability in delivery (reduced effects of humidification, bias flow, continuous v. breath-actuated) so as to achieve the same delivery (no matter what commercial ventilator system is used); (2) allow for maximal effects of breath actuation; and (3) allow for maximal effect to enhanced nebulizer efficiency using nebulizers having no dead volume.

The present invention is not limited to the precise configuration or nature of the circuit. In one or more embodiments, said circuit is a closed circuit. In other embodiments, said circuit is an open circuit.

Again, the present invention is not limited to particular vent configurations, or even to require a ventilator. In one or more aspects, said inspiratory and said expiratory lines are connected to a mechanical ventilator. In one or more aspects, said mechanical ventilator controls a breathing cycle, said cycle comprising an inspiration phase. In one or more aspects, the aerosol is administered during the inspiration phase of the breathing cycle. In other aspects, the aerosol is administered via an aerosol generator, suitable adapter, and patient interface (oral or nasal), and optionally, one or more valves.

Although the present invention has been described in considerable detail with regard to certain versions thereof, other versions are possible, and alterations, permutations and equivalents of the version shown will become apparent to those skilled in the art upon a reading of the specification and study of the drawings. For example, the relative positions of the elements in the aerosolization device may be changed, and flexible parts may be replaced by more rigid parts that are hinged, or otherwise movable, to mimic the action of the flexible part. In addition, the passageways need not necessarily be substantially linear, as shown in the drawings, but may be curved or angled, for example. Also, the various features of the versions herein can be combined in various ways to provide additional versions of the present invention. Furthermore, certain terminology has been used for the purposes of descriptive clarity, and not to limit the present invention. Therefore, any appended claims should not be limited to the description of the preferred versions contained herein and should include all such alterations and permutations that fall within the scope of the present invention.

Having now fully described this invention, it will be understood to those of ordinary skill in the art that the devices of the present invention can be used with a wide and equivalent range of conditions, formulations, and other parameters without departing from the scope of the invention or any embodiments thereof.

## Claims

1. An adapter (1200), comprising:
a housing forming a first channel (1265), a second channel (1260) and a cross channel (1215) providing a lumen between the first channel (1265) and the second channel (1260), the housing having a first end and a second end, wherein the first channel (1265) includes a first connector (1205) at the first end and a first tube (1256) at the second end, the first connector (1205) being adapted to be connectable to an inhalation line of a ventilator circuit, and wherein the second channel (1260) includes a second connector (1210) at the first end and a second tube (1257) at the second end, the second connector (1210) being adapted to be connectable to an exhalation line of the ventilator circuit;
the first channel (1265) comprising a first one-way valve (1270) to allow flow in a first direction and impair flow in a second direction, wherein the first one-way valve (1270) is positioned downstream, relative to the first direction, of the cross channel (1215);
the second channel (1260) comprising a second one-way valve (1290) to allow flow in a third direction and impair flow in a fourth direction, wherein the second one-way valve (1290) is positioned upstream, relative to the third direction, of the cross channel (1215); and
at least one of an aerosolization device and an aerosolization device port (1150) in the first channel (1265) positioned downstream, relative to the first direction, of the one-way valve (1270).

2. The adapter (1200) of claim 1, wherein a flow path through the first channel (1265) and a flow path through the second channel (1260) are designed such that an air pressure drop between the first end and the second end of the adapter (1200) is less than about 50cm H₂O at an air flow rate of 60 L/min.

3. The adapter (1200) of claim 2, wherein the first (1270) and second (1290) one-way valves do not invert when air pressure changes in an amount of up to about 140.6cm H₂O (2 psi) in less than 0.5 second.

4. The adapter of claim 2, wherein the air pressure drop ranges from about 0.05cm H₂O to about 10cm H₂O.

5. The adapter of claim 2, wherein the air pressure drop ranges from about 2cm H₂O to about 4cm H₂O.

6. The adapter of claim 1, further comprising:
a fluid accumulator (1242) for accumulating aerosolized fluid which condenses upstream of the second one-way valve in the second channel (1260) positioned upstream, relative to the third direction, of the second one-way valve (1290).

7. The adapter of claim 6, wherein the fluid accumulator (1242) comprises a port (1244).

8. The adapter of claim 7, wherein the port (1244) comprises a valve.

9. The adapter of claim 7, wherein the port (1244) comprises at least one member selected from a stop-cock, sphincter valve, injection site, and removable cap.

10. The adapter of claim 6, wherein the fluid accumulator (1242) comprises a reservoir (1246) formed in the second channel.

11. The adapter of claim 10, wherein the second channel (1260) has an oval cross-section.

## Patentansprüche

1. Adapter (1200), umfassend:
ein Gehäuse, welches einen ersten Kanal (1265), einen zweiten Kanal (1260) und einen Querkanal (1215) bildet, der ein Lumen zwischen dem ersten Kanal (1265) und dem zweiten Kanal (1260) bereitstellt, wobei das Gehäuse ein erstes Ende und ein zweites Ende aufweist, wobei der erste Kanal (1265) ein erstes Verbindungsstück (1205) an dem ersten Ende und ein erstes Rohr (1256) an dem zweiten Ende umfasst, wobei das erste Verbindungsstück (1205) dazu ausgebildet ist, mit einer Inhalationsleitung von einem Beatmungskreis verbindbar zu sein, und wobei der zweite Kanal (1260) ein zweites Verbindungsstück (1210) an dem ersten Ende und ein zweites Rohr (1257) an dem zweiten Ende aufweist, wobei das zweite Verbindungsstück (1210) dazu angepasst ist, mit einer Exhalationsleitung von dem Beatmungskreis verbindbar zu sein, wobei der erste Kanal (1265) ein erstes Einweg-Ventil (1270) umfasst, um eine Strömung in eine erste Richtung zu erlauben und eine Strömung in eine zweite Richtung zu behindern, wobei das erste Einweg-Ventil (1270) in Bezug auf die erste Richtung stromabwärts von dem Querkanal (1215) positioniert ist,
wobei der zweite Kanal (1260) ein zweites Einweg-Ventil (1290) umfasst, um eine Strömung in eine dritte Richtung zu erlauben und eine Strömung in eine vierte Richtung zu behindern, wobei das zweite Einweg-Ventil (1290) in Bezug auf die dritte Richtung stromaufwärts von dem Querkanal (1215) positioniert ist, und
eine Vernebler-Einrichtung oder/und ein Vernebler-Einrichtung-Anschluss (1150) in dem ersten Kanal (1265), die oder der in Bezug auf die erste Richtung stromabwärts von dem Einweg-Ventil (1270) positioniert ist.

2. Adapter (1200) nach Anspruch 1 wobei ein Strömungspfad durch den ersten Kanal (1265) und ein Strömungspfad durch den zweiten Kanal (1260) so ausgelegt sind, dass ein Luftdruck-Abfall zwischen dem ersten Ende und dem zweiten Ende von dem Adapter (1200) weniger als etwa 50 cm H₂O bei einer Luft-Strömungsrate von 60 L/min beträgt.

3. Adapter (1200) nach Anspruch 2, wobei das erste (1270) und das zweite (1290) Einweg-Ventil nicht invertieren, wenn sich Luftdruck um einen Betrag von bis zu etwa 140,6 cm H₂O (2psi) in weniger als 0,5 Sekunden ändert.

4. Adapter (1200) nach Anspruch 2, wobei der Luftdruck-Abfall in einem Bereich von etwa 0,05 cm H₂O bis etwa 10 cm H₂O liegt.

5. Adapter (1200) nach Anspruch 2, wobei der Luftdruck-Abfall in einem Bereich von etwa 2 cm H₂O bis etwa 4 cm H₂O liegt.

6. Adapter nach Anspruch 1, weiter umfassend:
einen Fluid-Sammler (1242) zum Sammeln vernebelten Fluids, welches stromaufwärts von dem zweiten Einweg-Ventil kondensiert, in dem zweiten Kanal (1260), positioniert stromaufwärts von dem zweiten Einweg-Ventil (1290) in Bezug auf die dritte Richtung.

7. Adapter nach Anspruch 6, wobei der Fluid-Sammler (1242) einen Anschluss (1244) umfasst.

8. Adapter nach Anspruch 7, wobei derAnschluss (1244) ein Ventil umfasst.

9. Adapter nach Anspruch 7, wobei der Anschluss (1244) wenigstens ein Element umfasst, das ausgewählt ist aus einem Absperrhahn, einem Sphinkter-Ventil, einer Injektionsstelle und einem entfernbaren Deckel.

10. Adapter nach Anspruch 6, wobei der Fluid-Sammler (1242) ein in dem zweiten Kanal ausgebildetes Reservoir (1246) umfasst.

11. Adapter nach Anspruch 10, wobei der zweite Kanal (1260) einen ovalen Querschnitt hat.

## Revendications

1. Adaptateur (1200) comprenant :
un boîtier formant un premier canal (1265), un second canal (1260) et un canal transversal (1215) fournissant une lumière entre le premier canal (1265) et le second canal (1260), le boîtier ayant une première extrémité et une seconde extrémité, dans lequel le premier canal (1265) comprend un premier connecteur (1205) au niveau de la première extrémité et un premier tube (1256) au niveau de la seconde extrémité, le premier connecteur (1205) étant adapté pour pouvoir être raccordé à une ligne d'inhalation d'un circuit de ventilateur, et dans lequel le second canal (1260) comprend un second connecteur (1210) au niveau de la première extrémité et un second tube (1257) au niveau de la seconde extrémité, le second connecteur (1210) étant adapté pour pouvoir être raccordé à une ligne d'expiration du circuit de ventilateur ;
le premier canal (1265) comprenant une première valve à une voie (1270) pour permettre l'écoulement dans une première direction et affaiblir l'écoulement dans une deuxième direction, dans lequel la première valve à une voie (1270) est positionnée en aval, par rapport à la première direction, du canal transversal (1215) ;
le second canal (1260) comprenant une seconde valve à une voie (1290) pour permettre l'écoulement dans une troisième direction et affaiblir l'écoulement dans une quatrième direction, dans lequel la seconde valve à une voie (1290) est positionnée en amont, par rapport à la troisième direction, du canal transversal (1215) ; et
au moins l'un parmi un dispositif d'aérosol et un orifice (1150) de dispositif d'aérosol dans le premier canal (1265) positionné en aval, par rapport à la première direction, de la valve à une voie (1270).

2. Adaptateur (1200) selon la revendication 1, dans lequel une trajectoire d'écoulement passant par le premier canal (1265) et une trajectoire d'écoulement passant par le second canal (1260) sont conçues de sorte qu'une chute de pression d'air entre la première extrémité et la seconde extrémité de l'adaptateur (1200) est inférieure à environ 50 cm H₂O à un débit d'air de 60 L/min.

3. Adaptateur (1200) selon la revendication 2, dans lequel les première (1270) et seconde (1290) valves à une voie ne s'inversent pas lorsque la pression d'air change selon une quantité allant jusqu'à environ 140,6 cm H₂O (2 psi) en moins de 0,5 seconde.

4. Adaptateur selon la revendication 2, dans lequel la chute de pression d'air va d'environ 0,05 cm H₂O à environ 10 cm H₂O.

5. Adaptateur selon la revendication 2, dans lequel la chute de pression d'air va d'environ 2 cm H₂O à environ 4 cm H₂O.

6. Adaptateur selon la revendication 1, comprenant en outre:
un accumulateur de fluide (1242) pour accumuler le fluide sous forme d'aérosol qui se condense en amont de la seconde valve à une voie dans le second canal (1260) positionné en amont, par rapport à la troisième direction, de la seconde valve à une voie (1290).

7. Adaptateur selon la revendication 6, dans lequel l'accumulateur de fluide (1242) comprend un orifice (1244).

8. Adaptateur selon la revendication 7, dans lequel l'orifice (1244) comprend une valve.

9. Adaptateur selon la revendication 7, dans lequel l'orifice (1244) comprend au moins un élément choisi parmi un robinet, une valve de sphincter, un site d'injection et un capuchon amovible.

10. Adaptateur selon la revendication 6, dans lequel l'accumulateur de fluide (1242) comprend un réservoir (1246) formé dans le second canal.

11. Adaptateur selon la revendication 10, dans lequel le second canal (1260) a une section transversale ovale.
